(19) Europäisches Patentamt
European Patent Office
Office européen des brevets

(11) Veröffentlichungsnummer: **0 001 774**
**A1**

(12) # EUROPÄISCHE PATENTANMELDUNG

(21) Anmeldenummer: **78101147.3**

(22) Anmeldetag: **14.10.78**

(51) Int. Cl.$^2$: **C 07 G 7/00**
A 61 K 37/64, C 07 G 7/02

(30) Priorität: **27.10.77 DE 2748295**

(43) Veröffentlichungstag der Anmeldung:
**16.05.79 Patentblatt 79/10**

(84) Benannte Vertragsstaaten:
**BE CH DE FR GB NL SE**

(71) Anmelder: **Bayer Aktiengesellschaft**
**Zentralbereich Patente,Marken und Lizenzen Bayerwerk**
**D-5090 Leverkusen 1(DE)**

(72) Erfinder: **Schnabel, Eugen, Dr.**
**Schimmelweg 6**
**D-5600 Wuppertal 11(DE)**

(72) Erfinder: **Reinhardt, Gerd, Dr.**
**Am Eckbusch 55a**
**D-5600 Wuppertal 1(DE)**

(72) Erfinder: **Schlumberger, Horst-Dieter, Prof. Dr.**
**Schwartnerstrasse 5**
**D-5600 Wuppertal 2(DE)**

(72) Erfinder: **Opitz, Hans-Georg, Dr.**
**Steinberger Weg 52**
**D-5600 Wuppertal 17(DE)**

(72) Erfinder: **Truscheit, Ernst, Dr.**
**Horather Strasse 160**
**D-5600 Wuppertal 1(DE)**

(72) Erfinder: **Tschesche, Harald, Prof. Dr.**
**Im Strohsiek 24**
**D-4800 Bielefeld 1(DE)**

(54) **Neue Derivate des BPTI(basic pancreatic trypsin-inhibitor), Verfahren zu ihrer Herstellung und ihre Verwendung als Arzneimittel.**

(57) Derivate des Kallikrein-Trypsin-Inhibitors und insbesondere des Kallikrein-Trypsin-Inhibitors aus Rinderorganen (BPTI = basic pancreatic trypsin inhibitor / Kunitz genannt) werden aus BPTI oder seinen Derivaten mit freien Carboxylgruppen durch Umsetzung mit nucleophilen Aminoverbindungen in Gegenwart von Carbodiimiden erhalten, gegebenenfalls anschließend desaminiert und/oder in Azoderivate übergeführt. Die BPTI-Derivate werden als Arzneimittel verwendet.

EP 0 001 774 A1

BAYER AKTIENGESELLSCHAFT 5090 Leverkusen, Bayerwerk

Zentralbereich
Patente, Marken und Lizenzen Er-kl

BEZEICHNUNG GEÄNDERT
siehe Titelseite

# Neue Derivate des BPTI, Verfahren zu ihrer Herstellung und ihre Verwendung als Arzneimittel

Die vorliegende Erfindung betrifft neue Derivate des Kallikrein-Trypsin-Inhibitors und insbesondere des Kallikrein-Trypsin-Inhibitors aus Rinderorganen (im folgenden BPTI = basic pancreatic trypsin inhibitor / Kunitz) genannt.

Es ist bereits bekannt geworden, daß BPTI [H. Kraut, E.K. Frey, E.Werle, Z. Physiol. Chem., 189, 97 (1930)], auch als Kunitz-Inhibitor bezeichnet [M. Kunitz, H.H. Northrop, J. Gen. Physiol. 19, 991 (1936)], eine Reihe von physiologischen bedeutsamen Enzymen, wie z.B. Kininogenine (Kininogenasen), Plasmin, Chymotrypsin und Trypsin hemmt (E. Werle in W. Brendel, G.L. Haberland: Neue Aspekte der Trasylol-Therapie 5, 9, F.K. Schattauer-Verlag, Stuttgart-New York 1972; H. Fritz, H. Tschesche, L.J. Greene, E. Truscheit (Hrsg.): Proteinase Inhibitors (Bayer Symposium V), Proc. 2nd International Research Conference, Springer-Verlag Berlin-Heidelberg-New York 1974).BPTI wird als Aprotinin (Freiname) zur Therapie und Prophylaxe von Schockzuständen sowie zur Prophylaxe postoperativer und postraumatischer Komplikationen eingesetzt.

Es ist weiterhin bekannt, daß die freien Carboxylgruppen von Proteinen in Gegenwart von wasserlöslichen Carbodiimiden mit Nukleophilen, insbesondere mit Aminen, unter Ausbildung von Carbonamidbindungen kondensiert werden können /D.G. Hoare und D.E. Koshland, J. Biol. Chem. 242, 2447 (1967)7.

Es ist außerdem bekannt, daß auf diese Weise auch im BPTI je nach den Umsetzungsbedingungen 3-4 der 5-Carboxylgruppen mit Glycinmethylester substituiert werden. /J. Chauvet und R. Acher, FEBS Letters 2,17 (1968)7. Weitere Umsetzungen von BPTI und Aminen in Gegenwart von Carbodiimiden sind in der Literatur nicht beschrieben worden.

Es wurde nun gefunden, daß man neue Derivate des BPTI bei welchen nur ein Teil der Carboxylgruppen modifiziert ist, erhält, wenn man den nativen BPTI oder Derivate des BPTI, welche freie Carboxylgruppen enthalten, bei pH-Werten zwischen 3 und 11 in wäßriger Lösung,welche gegebenenfalls auch organische Lösungsmittel und/oder Salze und/oder Denaturierungsmittel enthält, mit nucleophilen Aminoverbindungen, mit Ausnahme des Glycinäthylester, in Gegenwart von Carbodiimiden umsetzt, die erhaltenen Reaktionprodukte gegebenenfalls als solche isoliert und die Reaktionsprodukte gegebenenfalls desaminiert und/oder gegebenenfalls in entsprechende Azoderivate überführt.

Überraschenderweise besitzen die erfindungsgemäß erhältlichen neuen BPTI-Derivate ein breiteres und wertvolleres Inhibitionsspektrum als der literaturbekannte BPTI.

Die erfindungsgemäßen BPTI-Derivate hemmen beispielsweise im Gegensatz zu BPTI Elastasen aus Pankreas und Granulozyten. Gegenüber anderen Proteasen wie Chymotrypsin, Pankreas-Kallikrein, Plasma-Kallikrein, Plasmin, Kathepsin G und Trypsin unterschieden sich die Hemmspektren einzelner Derivate qualitativ und/oder quantitativ von dem des BPTI. Bei der Elastasehemmung handelt es sich nicht um die von J. Pütter und G.Schmidt-Kastner, Biochem. Biophys. Acta 127, 538 (1966) beschriebene Blockierung des Substrats, die nur bei hohen BPTI-Konzentrationen erfolgt, sondern um eine Hemmung des Enzyms.

Diese Elastase-Hemmwirkung ist umso überraschender, als aus dem derzeitigen Stand der Technik folgt /D.M. Blow, C.S. Wright, D. Kukla, A. Rühlmann, W. Steigemann und R. Huber, J. Mol. Biol. 69, 137 (1972); R. Huber, D. Kukla, W. Steigemann, J. Deisenhofer und A. Jones in H. Fritz, H. Tschesche, L.J. Greene und E.Truscheit (Hrsg.), Proteinase Inhibitors (Bayer Symposium V) Proc.2nd Intern. Res. Conference, S. 484, Springer-Verlag Berlin-Heidelberg-New York 1974/, daß BPTI substratartig mit seinem aktiven Zentrum (Lysin-15-Rest) in die Spezifitätstasche der hemmbaren Enzyme eingelagert wird und damit die Enzymaktivität blockiert.Selbst wenn die Seitenkette des Lysin-15-Restes bei einigen erfindungsgemäßen Derivaten desaminiert ist, ist nach dem derzeitigen Wissensstand nicht zu erwarten, daß sie in die relativ kleine Spezifitätstasche der Elastasen (D. Shotton in H. Fritz und H. Teschesche (Hrsg.),Proceedings of the International Research Conference on Proteinase Inhibitors, S. 47, Walter de Gruyter, Berlin-New York 1971) hineinpaßt.

Die Umsetzung von BPTI und den BPTI-Derivaten mit Carbodiimiden und nucleophilen Aminoverbindungen wird vorzugsweise bei pH-Werten zwischen 3 und 11, insbesondere zwischen pH 4 und pH 8,in wäßriger Lösung, die jedoch auch organische Lösungsmittel und/oder Salze und/oder Denturierungsmittel enthalten kann, durchgeführt. Als gegebenenfalls zusetzbare organische Lösungsmittel seien vorzugsweise genannt: Dimethylformamid, Dimethylsulfoxid, Hexamethylphosphorsäuretriamid, Pyridin, Dimethylacetamid. Als Salze seien genannt: LiCl, NaCl und als Denaturierungsmittel: Guanidin-Hydrochlorid, Harnstoff.

Als nucleophile Aminoverbindungen kommen vorzugsweise die folgenden Verbindungen bzw. Verbindungsklassen in Frage:

Ammoniak, Hydrazin, substituierte Hydrazine, primäre und sekundäre aliphatische oder cycloaliphatische Amine, welche alle gegebenenfalls noch zusätzliche funktionelle Gruppen wie z.B. Hydroxy, Alkoxy, Amino, Alkyl- und Dialkylamino, Mercapto, Alkylmercapto, Guanidino, Amidino, Carboxyl, Carboalkoxy, Carbamido, Cyano, Sulfo sowie auch aromatische oder heterocyclische Reste mit zusätzlichen funktionellen Gruppen u.a. tragen.
Besonders geeignete nucleophile Aminoverbindungen sind Aminozucker, Aminoalkohole, Aminosulfonsäuren, Derivate von Aminosäuren, wie z.B. Aminosäureester, -amide, -nitrile sowie mit den erforderlichen Schutzgruppen versehene Oligopeptide. Es kommen insbesondere solche Oligopeptide in Betracht, die Asparaginsäure- und/oder Glutaminsäure-Reste in Peptid- und/oder Isopeptid-Bindung enthalten.

Die vorgenannten Aminosäure- und Peptidderivate können aus den optisch aktiven Aminosäuren oder aus deren Racematen hergestellt werden. Verwendet man die tertiären Butylester der vorgenannten Aminosäuren oder Peptide, so können deren Carboxylgruppen nach der Umsetzung mit dem BPTI oder den in Frage kommenden BPTI-Derivaten durch Behandeln mit Säure nach an sich bekannten Methoden freigelegt werden.

Bei der Herstellung der erfindungsgemäßen neuen BPTI-Derivate vorzugsweise einsetzbare Aminoverbindungen besitzen die folgende Struktur:

$$\begin{array}{c}H-N-X\\ \quad | \\ \quad Y\end{array} \qquad (I)$$

In dieser Formel bedeuten:

X H oder einen unverzweigten oder einen verzweigten Alkylrest oder einen Cycloalkylrest oder einen Arylrest. Diese Reste können gegebenenfalls zusätzliche Substituenten tragen.

Y Dieselben Gruppen wie bei X. Wenn X = H ist, steht Y insbesondere für einen Rest der allgemeinen Formel

$$-\left(\begin{array}{c}A\\ |\\ C\\ |\\ B\end{array}\right)_n-Z \qquad (II)$$

in der n die Werte 1 bis 5 besitzt und A Wasserstoff oder einen Alkylrest darstellt, während B ebenfalls Wasserstoff oder einem Alkylrest entspricht, der gegebenenfalls mit einer Hydroxyl-, Amino-, Mercapto- oder Guanidinogruppe oder einem Heterocyclus oder auch durch eine

Gruppe der Bedeutung Z substituiert ist, wobei Z hier und in Formel (II) für die folgenden funktionellen Gruppen stehen kann:

$$-CH_2OH,\ -CH_2-NH_2,\ -\underset{NH_2}{C}{=}NH,\ -CN,\ -CO-\underset{Z}{N}-X,\ -CO-OX$$

wobei X und Z wiederum die Bedeutungen wie in Formeln (I) und (II) haben, aber Z nicht $-CO-OC_2H_5$ sein darf, sofern in (II) A = H und B = H und n = 1 sind. - Bei asymmetrisch substituierten Derivaten können die optisch aktiven oder auch die racemischen Verbindungen eingesetzt werden.

Weiterhin kann Y auch eine Gruppe der Formel $-(CH_2)_m-SO_3H$ mit m = 1 bis 4 sein.

Darüber hinaus kann Y zusammen mit X in Formel (I) ein gemeinsames 3- bis 6-gliedriges Ringsystem bilden, das gegebenenfalls weitere Heteroatome enthalten kann und/oder auch Substituenten des Typs Z tragen kann.

Ferner kann Y auch einer Gruppierung der allgemeinen Formel

$$-\underset{R'}{N}-R \qquad (III)$$

entsprechen, wobei R und R' die gleiche Bedeutung wie X in Formel (I) haben können.

Als Carbodiimide können bei der erfindungsgemäßen Umsetzung zur Herstellung der erfindungsgemäßen Verbindungen eine Reihe von organischen Carbodiimiden eingesetzt werden, insbesondere folgende Carbodiimide haben sich als für die Durchführung des erfindungsgemäßen Verfahrens besonders geeignet herausgestellt: Dicyclohexylcarbodiimid, N-Cyclohexyl-N'-[2-(4-morpholinyl)-äthyl]-carbodiimid-metho-toluol-4-sulfonat, N-Äthyl-N'-(3-Dimethylamino-propyl)-carbodiimid-hydrochlorid,

N-tert.-Butyl-N'-(3-dimethylamino-propyl)-carbodiimid,
N-Cyclohexyl-N'-(3-dimethylaminopropyl)-carbodiimid-Hydrochlorid,
N-Isopropyl-N'-(3-dimethylaminopropyl)-carbodiimid-Hydrochlorid,
N-Cyclohexyl-N'-(3-dimethylaminopropyl)-carbodiimid-metho-toluol-4-sulfonat,
N,N'-Diisopropyl-carbodiimid,
N,N'-Di-para-tolyl-carbodiimid.

Die erfindungsgemäße Umsetzung erfolgt bevorzugt bei Temperaturen zwischen -20°C und +50°C, insbesondere zwischen 0°C und 30°C.

Pro Mol BPTI bzw. BPTI-Derivat werden 10 - 100 Mol, vorzugsweise 10 bis 25 Mol der nucleophilen Aminoverbindung eingesetzt. Bezogen auf die nucleophile Aminoverbindung wird das verwendete Carbodiimid im Unterschuß eingesetzt. Vorzugsweise werden ca. 85 - 95 % der molaren Menge der nucleophilen Aminoverbindung eingesetzt.

Bei den Umsetzungen resultierende Gemische können gegebenenfalls nach an sich bekannten Verfahren, z.B. durch Elektrophorese, Gelfiltration oder Ionenaustauschchromatographie fraktioniert werden.

Weiterhin erhält man erfindungsgemäß neue Derivate des BPTI dadurch, daß man die durch Umsetzung von BPTI oder seinen Derivaten, welche freie Carboxylgruppen enthalten, mit Carbodiimiden und den nucleophilen Aminoverbindungen gewonnenen Derivate anschließend ganz oder teilweise desaminiert. Dazu werden die Derivate mit Nitrosylionen-liefernden Agentien wie Salzen, Estern oder gemischten Anhydriden der salpetrigen Säure in saurer Lösung gegebenenfalls in Gegenwart von Zusatzstoffen im heterogenen oder homogenen System umgesetzt.

Alternativ kann man die nachträgliche Desaminierung auch durch Umsetzen der Derivate mit geeigneten nicht kuppeln-



BAD ORIGINAL

den Diazoniumverbindungen durchführen (z.B. durch Umsetzung mit dem Reaktionsprodukt aus 5-Aminotetrazol und Nitrosylionen liefernden Verbindungen).

Weiterhin erhält man erfindungsgemäß neue Derivate des BPTI dadurch, daß man die durch Umsetzung von BPTI oder seinen Derivaten mit freien Carboxylgruppen, mit Carbodiimiden und nucleophilen Aminoverbindungen gewonnenen Derivate anschließend mit Diazoniumverbindungen in wäßriger Lösung, die gegebenenfalls auch organische Lösungsmittel, wie beispielsweise Pyridin, Chinolin, Dimethylformamid, Dimethylsulfoxid oder Hexamethylphosphorsäuretriamid, und/oder Zusätze, wie Salze, beispielsweise Guanidinhydrochlorid oder Lithiumchlorid, und/oder Nichtelektrolyte, wie z.B. Harnstoff, enthalten kann, zu Azo-BPTI-Derivaten kuppelt.

Die Kupplungsreaktionen werden je nach der Reaktivität der Diazoniumverbindungen bei pH-Werten zwischen 1 und 12 vorzugsweise bei pH 5 bis 11 durchgeführt, wobei Lösungen des BPTI oder von BPTI-Derivaten in Wasser oder in den oben aufgeführten Lösungsmitteln, die ggf. die oben angegebenen Zusatzstoffe enthalten, mit den Diazoniumsalzlösungen vereinigt werden. Der pH-Wert des Reaktionsgemisches wird durch Zugabe von Alkalimetall- bzw. Erdalkalimetall-Oxiden, -Hydroxiden, -Carbonaten, -Hydrogencarbonaten oder tert. Aminen, wie z.B. Triäthylamin, N-Methylmorpholin, Triäthanolamin oder Pyridin, eingestellt und konstant gehalten. Die Umsetzungen können aber auch in Pufferlösungen, insbesondere Phosphat- oder Boratpuffern durchgeführt werden. Die Umsetzungsgeschwindigkeiten können zweckmäßig über den pH-Wert des Reaktionsgemisches gesteuert werden.

Der Endpunkt der Umsetzung kann beispielsweise durch Tüpfelreaktion ermittelt werden, bei der eine Probe der Reaktionslösung mit 1-(2-Aminoäthylamino)-naphthalin als Kupplungspartner umgesetzt wird.

Während der Kupplungsreaktionen hält man die Temperaturen der Reaktionsgemische bei -15°C bis 30°C, vorzugsweise bei 0°C bis 10°C. Unter Umständen ist es zweckmäßig, die Kupplung zunächst bei Temperaturen zwischen 0°C und 4°C durchzuführen und anschliessend die Temperatur bis auf 25°C zu erhöhen. Überschüssige Diazoniumverbindung kann ggf. vor der Aufarbeitung des Reaktionsgemisches durch Zusetzen von z.B. von Phenol zur Reaktionslösung gebunden werden. Der Verbrauch an Diazoniumverbindung während der Kupplungsreaktionen kann anhand von Probekupplungen mit 1-(2-Aminoäthylamino)-naphthalin durch Tüpfeln verfolgt werden. Das molare Verhältnis von Diazoniumverbindungen zu eingesetztem BPTI-Derivat beträgt 1:1 bis 10:1, vorzugsweise 1:1 bis 5:1, wobei die Konzentrationen an BPTI-Derivaten in den zur Kupplung verwendeten Lösungen bei 1 - 30 %, vorzugsweise bei 5 - 15 % liegen.

Als Azokomponenten sind zur Darstellung der erfindungsgemäßen Azo-BPTI-Derivate ein- oder mehrkernige aromatische sowie heterocyclische Diazoniumverbindungen geeignet, die aus den entsprechenden Aminen dargestellt werden. Diese können noch einen oder mehrere Substituenten tragen. Insbesondere sind Derivate des Anilins geeignet, z.B. Alkylaniline, Alkoxyaniline, Halogenderivate des Anilins, Nitroaniline, Cyanoaniline, Trifluormethylaniline, Acetaminoaniline, Amino-acetophenone, Aminobenzolsulfonsäuren, Anilindisulfonsäuren, Aminobenzolarsinsäuren, Aminobenzoesäuren, Aminodiphenyläther; als mehrkernige Amine sind besonders α- und ß-Naphthylamin sowie deren Derivate, z.B. Aminonaphthalinsulfonsäuren, geeignet. Als Heterocyclen eignen sich insbesondere Aminotriazol und Aminophenyltriazole, die auch substituiert sein können.

Die Diazotierung der bei der erfindungsgemäßen Azokupplung eingesetzten Amine wird nach den üblichen Verfahren [R.Pütter, in "Houben-Weyl, Methoden der organischen Chemie", Band 10/3, S. 1 ff., Hrsg. E.Müller unter Mitwirkung von O.Bayer, H.Meerwein und K.Ziegler, Georg Thieme Verlag, Stuttgart 1965] durch Umsetzen mit Nitrosylionen-liefernden Verbindungen, insbesondere Natriumnitrit in mineralsaurer Losung oder Suspension ggf. in Anwesenheit von organischen Lösungsmitteln, insbesondere von Dimethylformamid, Essigsäure oder Propionsäure, bei Temperaturen von -20°C bis 50°C, vorzugsweise -5°C bis 20°C, oder aber mit Nitrosylschwefelsäure bzw. Nitrosylchlorid in Essigsäure-Propionsäure-Gemischen unter Zusatz von Mineralsäure bei Temperaturen um vorzugsweise -20° bis 0° durchgeführt. Die Zeitdauer der Diazotierung ist sehr unterschiedlich; sie hängt ab von der Löslichkeit der Amine sowie deren Substitutionsmuster und schwankt zwischen wenigen Minuten und mehreren Stunden. Überschüssige Nitrosylionen werden nach der Diazotierung durch Umsetzen mit Amidosulfonsäure oder Harnstoff zerstört, um eine Desaminierung der BPTI-Derivate durch Nitrosylionen, wie sie beim Durchführen der Kupplungen im saurem Milieu möglich ist,zu verhindern.

Bei der Kupplung von BPTI-Derivaten mit den Diazoniumverbindungen werden Gemische verschiedener Komponenten erhalten. Die Zusammensetzung dieser Azo-BPTI-Derivat-Gemische kann durch die Wahl der Umsetzungsbedingungen, insbesondere über das Verhältnis von zur Kupplung eingesetztem BPTI-Derivat und Diazoniumverbindung, den pH-Wert der Reaktionsgemische, die Zeitdauer der Umsetzung sowie durch die obengenannten Zusätze zu den Reaktionslösungen gesteuert werden.

Weiterhin erhält man erfindungsgemäß neue Derivate des BPTI dadurch, daß man die durch Umsetzung von nucleophilen Aminoverbindungen und Carbodiimiden mit BPTI- oder BPTI-Derivaten erhaltenen Produkte nachträglich entweder mit Tetranitromethan

(B. Meloun, J. Fric, F. Sorm; Eur. J. Biochem. 4, 112 (1968)) oder erfindungsgemäß mit Salpetersäure nitriert oder aber in der eben beschriebenen Weise mit Diazoniumverbindungen kuppelt, daraufhin nach an sich bekannten Verfahren (M. Sokolowsky, J.F. Riordan, B.L. Vallee; Biochem. Biophys. Res. Comms. 27, (1967)) die eingeführten Nitro- bzw. Azogruppen zu Aminogruppen reduziert, weiterhin die daraus resultierenden Derivate des BPTI, die nun an den Tyrosinresten 10 und/oder 21 zusätzliche Aminogruppen in o-Stellung zur phenolischen Hydroxylgruppe tragen, nach üblichen Verfahren diazotiert und letztlich mit Phenolen oder aromatischen Aminen oder heterocyclischen Verbindungen kuppelt.

Wie bereits oben erwähnt, werden als Ausgangsprodukte zur Herstellung der erfindungsgemäßen Verbindungen BPTI und BPTI-Derivate, die freie Carboxylgruppen enthalten, eingesetzt.

Als Ausgangsprodukte seien neben dem BPTI beispielhaft genannt:

1) Desamino-BPTI-Derivate, welche ganz oder teilweise desamidiert sein können und/oder die an einem der beiden Tyrosinreste 10 oder 21 in ortho-Stellung zur phenolischen Hydroxylgruppe eine Nitrogruppe tragen; sie können auch am Tyrosinrest 10 und/oder 21 in ortho-Stellung zur phenolischen Hydroxylgruppe eine Aminogruppe enthalten. Man erhält sie aus BPTI oder Derivaten des BPTI nach einem der folgenden Verfahren:

   **a) Umsetzung derselben in saurer Lösung mit Nitrosylionen liefernden Agentien wie beispielsweise Alkali- oder Erdalkalinitriten, Estern der salpetrigen Säure oder gemischten Anhydriden der salpetrigen Säure, gegebenenfalls in Gegenwart von Zusatzstoffen, beispielsweise solchen, die die Konformation von Proteinen beeinflussen (z. B. organischen Lösungsmitteln, Detergentien oder Guanidiniumhy-**

drochlorid) in heterogenem oder in homogenem System und Aufarbeitung der Reaktionsgemische nach an sich bekannten Methoden. Man erhält dabei Gemische von BPTI-Derivaten unterschiedlichen Desaminierungsgrades, von denen ein Teil auch an den Tyrosinresten 10 und/oder 21 nitriert worden ist. Diese Nitrierung läßt sich zurückdrängen, wenn man während der Desaminierungsreaktion unter Schutzgas oder Evakuierung arbeitet und/oder einen Akzeptor für Elektrophile, z. B. Phenol, zusetzt. Während der Aufarbeitung kann die Nitrierung durch Alkalisieren des Ansatzes verhindert werden.

b) Umsetzung derselben in saurer, neutraler oder schwach alkalischer Lösung mit Diazoniumverbindungen, die man durch Diazotierung von heterocyclischen Aminen, insbesondere von 3-Amino-1,2,4-triazol oder 5-Aminotetrazol, erhält und Aufarbeitung des Reaktionsgemisches nach an sich bekannten Methoden. Den Reaktionsansätzen können gegebenenfalls Zusatzstoffe, wie z. B. organische Lösungsmittel, Detergentien, Guanidiniumhydrochlorid beigegeben werden. Man erhält dabei Gemische von BPTI-Derivaten unterschiedlichen, im Vergleich zu Variante a) im wesentlichen geringeren, Desaminierungsgrades, aus deren Absorptionsspektren ersichtlich ist, daß weder deren Tyrosinreste zu Azo-Derivaten noch deren primäre Aminogruppen zu Triazenen umgewandelt worden sind.

2) Desamido-BPTI, hergestellt durch längeres (20- bis 30-tägiges) Stehenlassen von nativem BPTI in wäßriger Säure (vorzugsweise 2n Salzsäure) bei Raumtemperatur.

3) Mononitro-BPTI erhalten nach B. Meloun et. al. Europ. J. Biochem. 4, 112 (1968) durch direkte Nitrierung von BPTI oder erfindungsgemäß mit Salpetersäure.

4) Dinitro-BPTI ebenfalls erhalten nach B. Meloun et. al. Europ.J. Biochem. 4, 112 (1968) oder erfindungsgemäß mit Salpetersäure.

5) Derivate des BPTI, die an den Tyrosinresten 10 und/oder 21 Aminogruppen tragen und aus den Derivaten gemäß 3) und 4) durch Reduktion nach an sich bekannten Methoden z.B. nach M. Sokolovsky et.al. Biochem. Biophys. Res. Comm. 27, 20 (1967).

6) Guanidierter BPTI,erhalten nach B. Kassell et.al. Biochemistry 5, 3449 - 3453.

7) Citraconylierter BPTI,erhalten aus BPTI durch Umsetzung mit Citraconsäureanhydrid u.a.

Die erfindungsgemäßen BPTI-Derivate sind neu. Sie lassen sich durch chemische, physikalisch-chemische, biochemische sowie biologische Eigenschaften charakterisieren und gegenüber bekannten Substanzen abgrenzen. Es wurden folgende Kriterien herangezogen:

## 1.) Aminosäurezusammensetzung

Die Aminosäurezusammensetzung wurde nach S.Moore, D.H.Spackman, W.H.Stein, Anal.Chem. 30, 1185 (1958) bestimmt. Die Gehalte charakteristischer Aminosäuren sind für eine Reihe der erfindungsgemäßen Carboxyl-modifizierter BPTI-Derivate in den betreffenden Beispielen angegeben. Tabelle 1 gibt die Gehalte charakteristischer Aminosäuren für einige weitere Carboxyl-modifizierte BPTI-Derivate wieder. Weiterhin ist aus der Tabelle ersichtlich, wie sich der Gehalt dieser Aminosäuren nach einer anschliessenden Kupplungs- oder Desaminierungsreaktion verändert hat.

2.) Elektrophoretische Eigenschaften

Cellogelelektrophoresen: Für die Cellogelelektrophoresen wurden 2.5 cm breite Celluloseacetatstreifen der Fa. Serva unter den üblichen Bedingungen /J.Kohn, Cellulose Acetate Electrophoresis and Immuno-Diffusion Techniques in I.Smith, Chromatographic and Electrophoretic Techniques, Vol. II, W.Heinemann, Ltd., London and Interscience Publishers 56-90 (1960);*/Gelman Instrument Company (1968) / verwendet. Der eingesetzte Pyridin-Acetat-Puffer pH 6.05 wurde durch Auffüllen eines Gemisches von 200 ml Pyridin und 20 ml Eisessig mit Wasser auf 2 Ltr. erhalten. Die Trennungen wurden bei 150 V mit einer Stromstärke von 0.8 mA / cm Streifenbreite durchgeführt, wobei man stets zum Vergleich BPTI als Standard mitlaufen ließ. Zum Anfärben der Streifen wurde eine Lösung von 5 g Amidoschwarz in einer Mischung von 900 ml Methanol und 100 ml Eisessig verwendet.

Disk-Elektrophoresen: Die Disk-Elektrophoresen wurden in einem 15 %igen Trenngel mit 0.03 m Imidazol-Puffer pH 7.1 als Anodenpuffer und 0.03 m $\epsilon$-Aminocapronsäure, 0.01 m Imidazol-Puffer vom gleichen pH-Wert als Kathodenpuffer durchgeführt. Die Stromstärke betrug 2.5 mA pro Röhrchen; die Trennung wurde beendet, sobald der Markerfarbstoff (Methylgrün) vollständig durch das

*) Gelman Procedures, Techniques, and Apparatus for Electrophoresis,

**Tabelle 1** Für einige Carboxyl-modifizierte BPTI-Derivate durch Aminosäureanalyse ermittelte Gehalte an charakteristischen Aminosäuren (Mol/Mol Derivat) 1)

| BPTI-Derivat nach Beispiel | hergestellt aus BPTI-Derivat nach Beispiel | Gly 2) | Ala 2) | Tyr | Lys | Arg | X (AS) 3) |
|---|---|---|---|---|---|---|---|
| BPTI 4) | - | 6.09 | 6.00 | 3.89 | 4.08 | 6.01 | |
| 2 | - | 5.95 | 6.00 | 3.91 | 4.02 | 5.98 | |
| 46 | 2 | 5.95 | 5.94 | 3.97 | 0.25 | 4,82 | |
| 48 | 2 | 6.00 | 6.60 | 3.67 | 2.17 | 5.19 | |
| 50 | 2 | 5.85 | 6.10 | 1.90 | 3.90 | 5.66 | |
| 65 | 2 | 5.40 | 6.13 | 2.86 | 3.83 | 5.74 | |
| 6 | - | 5.74 | 6.00 | 3.52 | 4.00 | 6.00 | 7.49 (Glu) |
| 57 | 6 | 6.00 | 6.63 | 2.44 | 2.49 | 5.26 | 6.86 (Glu) |
| 15 | - | 6.06 | 6.00 | 3.74 | 4.14 | 6.16 | 6.36 (Phe) |
| 54 | 15 | 6.00 | 5.62 | 1.85 | 2.05 | 5.10 | 5.85 (Phe) |
| 18 | - | 6.36 | 5.40 | 3.69 | 3.84 | 5.75 | 1.82 (Ser) |
| 68 | 18 | 5.88 | 6.01 | 1.51 | 3.67 | 5.62 | 1.84 (Ser) |
| 27 | - | 5.92 | 6.00 | 3.73 | 4.05 | 8.26 | |

**Tabelle 1** Fortsetzung

| BPTI-Derivat nach Beispiel | hergestellt aus BPTI-Derivat nach Beispiel | Gly 2) | Ala 2) | Tyr | Lys | Arg | X (AS) 3) |
|---|---|---|---|---|---|---|---|
| 58 | 27 | 5.32 | 6.05 | 3.01 | 3.71 | 8.12 | |
| 29 | - | 6.60 | 6.15 | 3.43 | 3.92 | 6.06 | 3.93 (His) |
| 56 | 29 | 6.00 | 6.00 | 2.44 | 3.20 | 5.07 | 1.04 (His) |
| 30 | - | 6.22 | 5.73 | 3.69 | 3.77 | 5.03 | 3.84 (Thr);5,75 (I |
| 67 | 30 | 6.30 | 5.70 | 1.72 | 3.58 | 5.40 | 4.00 (Thr);5.11 (I |
| 38 | - | 6.10 | 5.76 | 3.92 | 3.95 | 5.65 | 5.84 (Glu) |
| 72 | 38 | 6.00 | 6.00 | 1.91 | 4.00 | 5.68 | 5.86 (Glu) |

1) Das Molekulargewicht der Derivate wurde wie für BPTI zu 6511 angenommen.

2) Der Gly- bzw. Ala-Gehalt dient als interner Standard.

3) Werte für die durch die Carboxylmodifizierung eingeführten Aminosäuren; in Klammern ist die betreffende Aminosäure aufgeführt.

4) Zum Vergleich wurden die Werte der Aminosäuren für BPTI in die Tabelle aufgenommen. Die theoretischen Werte betragen: Gly, Ala und Arg je 6, Tyr und Lys je 4.

Gel hindurchgewandert war. Nach der Trennung wurden die Gele in einer 0.1 %igen Lösung von Amidoschwarz in 7 %iger Essigsäure 30 Minuten lang gefärbt. Überschüssiger Farbstoff wurde anschließend mit 7 %iger Essigsäure wieder entfernt.

In den Beispielen ist als relative elektrophoretische Beweglichkeit jeweils der Quotient aus der Wanderungsstrecke des betreffenden BPTI-Derivates und der Wanderungsstrecke des BPTI angegeben. Das Vorzeichen gibt die Wanderungsrichtung an. Negatives Vorzeichen bedeutet also kathodische Beweglichkeit.

3.) Absorptions-Spektren

Zur weiteren Charakterisierung der erfindungsgemäßen BPTI-Derivate eignen sich besonders bei den Azo- und/oder Nitrogruppen enthaltenden Verbindungen die Absorptionsspektren. Charakteristische Daten sind für einige Derivate in den betreffenden Beispielen angegeben.

4.) Proteasen-Inhibitionsspektren

a) Elastase-Inhibition

α) Pankreas-Elastase-Inhibition

Für die Hemmversuche mit den erfindungsgemässen BPTI-Derivaten wurde kristallisierte Pankreas-Elastase (Schwein) der Fa. Nutritional Biochemicals Corp. verwendet.. Als Substrate wurden Elastin-Kongorot/M.A. Naughton und F.Sanger, Biochem.J. 78, 156 (1961)/, lösliches Elastin /S.Keller und I.Mandl, Biochem.Med. 5, 342 (1971)/ sowie besonders vorteilhaft Succinyl-L-alanyl-L-alanyl-L-alanin-p-nitroanilid /J.Bieth, B.Spiess und C.G.Wermuth, Biochem.Med. 11, 350 (1974)/ verwendet. Das synthetische Peptidsubstrat ermöglicht eine einfache colorimetrische Bestimmung der im Test eingesetzten Enzymaktivität. Der Elastase-Einsatz betrug im

Elastin-Kongorot-Test ca. 1,1 nkat (vgl. Enzyme Nomenclature, Recommendations /1972/ of the International Union of Pure and Applied Chemistry and the International Union of Biochemistry, 1973, Elsevier Amsterdam-New York, S. 26-27); mit löslichem Elastin als Substrat und ebenso mit Succinyl-L-alanyl-L-alanyl-L-alanin-p-nitroanilid wurden ca. 0.25 nkat Elastase pro Test-Ansatz eingesetzt. Zur quantitativen Bestimmung der Elastase-Hemmung wurden die oben angegebenen Enzym-Mengen mit Inhibitorlösungen definierter Konzentration versetzt und die Substratlösung zugegeben. Um maximale Komplexbildung sicherzustellen, wurden in einigen Fällen Enzym und Inhibitor vor der Zugabe des Substrats 15 Min. vorinkubiert.

Die Hydrolyse des Substrats wurde beim Elastin-Kongorot-Test durch Messung der Extinktion bei 492 nm der nach einer definierten Zeit gebildeten löslichen Spaltprodukte bestimmt. Beim Test mit löslichem Elastin wurde die Hydrolyse-Rate durch photometrische Bestimmung der nach einer definierten Zeit in Lösung gegangenen mit Ninhydrin färbbaren Spaltprodukte ermittelt. Bei Verwendung von Succinyl-L-alanyl-L-alanyl-L-alanin-p-nitroanilid als Substrat wurde die Hydrolyse durch kontinuierliche Messung der Extinktion des freigesetzten p-Nitroanilins bei 410 nm bestimmt. Die Hemmwerte (ausgedrückt in % Hemmung) wurden ermittelt, indem man die nach Inhibitorzusatz gemessene Elastase -Restaktivität von der Aktivität der Enzymkontrolle substrahierte. Die Hemmwerte einiger erfindungsgemässer BPTI-Derivate sind in Tabelle 2 zusammengestellt.

β) Granulozyten-Elastase-Inhibition

Das für die Hemmteste verwendete Isoenzymgemisch wurde nach K.Ohlsson und I.Olsson /Europ.J.Biochem. 42, 519 (1974)/ aus Humangranulozyten gewonnen. Als Substrat ist besonders Succinyl-L-alanyl-L-alanyl-L-alanin-p-nitroanilid /J.Bieth, B.Spiess und C.G.Wermuth, Biochem.Med. 11, 350 (1974)/

geeignet. Die Hemmwerte einiger BPTI-Derivate sind in Tabelle 2 aufgenommen; der Enzym-Einsatz betrug 0.25 nkat pro Test. Die Hemmwerte sind Absolutwerte und nicht auf BPTI bezogen. Sie wurden ermittelt, wie für Pankreas-Elastase angegeben.

b) Chymotrypsin-Inhibition

Bei der Bestimmung der Aktivität von Chymotrypsin diente Succinyl-L-phenylalanin-ß-naphthylester in einem 0.25 m Trispuffer pH 7.2, der 5 m M an Calciumchlorid war, als Substrat. Das bei der Spaltung gebildete ß-Naphthol wurde photometrisch bei 329 nm bestimmt.

2 mg kristallisiertes α-Chymotrypsin wurden in 5 ml 0.001n-Salzsäure, die 5 m M an Calciumchlorid war, gelöst und 0.5 ml dieser Lösung mit 0.25 m Trispuffer pH 7.2 (s.oben) auf 10 ml aufgefüllt. 50 µl der so erhaltenen Enzymlösung fügte man zu 2.35 ml des 0.25 m Trispuffers und versetzte diese Lösung für die Enzymkontrolle mit 100 µl Wasser, für den Inhibitionstest jedoch mit 100 µl einer BPTI-Derivatlösung mit bekanntem Gehalt. Nach gutem Durchmischen und einer Äquilibrierphase von 15 Minuten oder ggf. auch ohne Vorinkubation setzte man 25 µl der Substratlösung zu. Die Substratlösung wurde hergestellt durch Lösen von 39.1 mg Succinyl-L-phenylalanin-ß-naphthylester in 1 ml Dimethylsulfoxid. Die durch den Inhibitor bewirkte prozentuale Hemmung errechnet sich nach der folgenden Gleichung:

$$\frac{1 - \Delta OD\ Hemmansatz}{\Delta OD\ Kontrolle} \cdot 100 = \%\ Hemmung$$

wobei die optische Dichte um den durch Spontanhydrolyse des Naphthylesters bedingten ß-Naphthol-Anteil korrigiert wurde.

Die in der Tabelle 2 für einige BPTI-Derivate angegebenen Hemmwerte sind auf die durch die gleiche Menge BPTI bewirkte Hemmung (= 100 %) bezogen.

c) Cathepsin G - Inhibition

Das Enzym wurde nach einem kombinierten Verfahren nach J.R.Baugh und J.Travis /Biochemistry 15, 836 (1976)/ und nach W.Schmidt und K.Havemann /Z.Physiol.Chem. 355, 1077 (1974) isoliert. Die Bestimmung der Aktivität erfolgte, wie für Chymotrypsin beschrieben, mit Succinyl-L-phenylalanin-ß-naphthylester als Substrat in einem 0.25 m Trispuffer pH 7.2, der 0.01 % Brij (R) und 0.005 Mol/l Magnesiumchlorid enthielt. Der Enzym-Einsatz betrug 0.25 nkat pro Test. Die Berechnung der prozentualen Hemmung wurde, wie unter b) beschrieben, durchgeführt.

d) Kininogenasen-Inhibition

α) Plasma-Kininogenasen-Inhibition

Die Kininogenasen-Aktivität wurde nach der Deutschen Offenlegungsschrift 25 27 932 vom 22.1.76, Erfinder L.G.Svendsen, Pentapharm AG., Basel, mit Benzoyl-L-prolyl-L-phenylalanyl-L-arginin-p-nitroanilid als Substrat bestimmt mit einem Einsatz von 0.1 nkat Enzym je Test. Das Gemisch der Isoenzyme wurde nach C.Sampaio et al. /Arch.Biochem.Biophys. 165, 133 (1974)/ durch Affinitätschromatographie aus der Human-Plasmafraktion Cohn IV-1 isoliert. Die in Tabelle 2 für einige BPTI-Derivate aufgeführten Hemmwerte wurden, wie unter Chymotrypsin-Inhibition angegeben, ermittelt.

ß) Pankreas-Kininogenase-Inhibition

Das Testenzym wurde nach C.Kutzbach und G.Schmidt-Kastner /Z.Physiol.Chem. 353, 1099 (1972)/ erhalten, und die Aktivitätsbestimmungen wurden nach denselben Autoren (s.o.) durchgeführt. Der Enzym-Einsatz betrug 10 nkat pro Test. Die in Tabelle 2 für einige BPTI-Derivate aufgeführten Hemmwerte wurden, wie unter Chymotrypsin-Inhibition angegeben, ermittelt.

e) Plasmin-Inhibition

Die enzymatische Aktivität von Plasmin wurde mit Azokasein bestimmt. Als Enzym wurde mit Urokinase aktiviertes Plasminogen benutzt, das mittels Affinitätschromatographie /D.G.Deutsch, E.T.Merz; J.Med. 3, 224 (1972)/ aus Humanplasma gewonnen wurde.

Die Herstellung des Substrates und das Prinzip des Testes sind in der Arbeit von P.M.Starkey, A.J.Barrett; Biochem.J. 155, 255 (1976) beschrieben. Man bereitet für jede Bestimmung zwei Ansätze A und B mit je 0.6 CU Plasmin, gelöst in 1.0 ml Puffer (0.1 m Tris-(hydroxymethyl)-aminomethan, 0.05 M NaCl, eingestellt auf pH 7.2 mit HCl). Beide Ansätze erhalten je 1.25 µg BPTI bzw. BPTI-Derivat, gelöst in 1.25 ml Puffer (s.o.); zum Vergleich dient je ein Ansatz A und B mit 1,25 ml Puffer anstelle des Inhibitors. Man inkubiert alle Ansätze 10 Min. bei 37°C und gibt darauf je 0,45 ml einer 2 %igen Azokaseinlösung (in Puffer, s.o.) zu. Serie A versetzt man direkt danach mit 0,3 ml 30 %iger Trichloressigsäurelösung (TCA); Serie B wird 60 Min. bei 37°C inkubiert und darauf mit 0,3 ml TCA versetzt. Die Ansätze werden nach ca. 20 Min. zentrifugiert; die Extinktionen der Überstände werden photometrisch bei 340 nm in einer 1 cm-Küvette bestimmt. Als Maß der enzymatischen Aktivität gilt die Extinktionsdifferenz der parallelen Ansätze A und B ($\Delta$ E).

Bezeichnet man die Extinktionsdifferenz, die von Plasmin allein hervorgerufen wird, mit $\Delta E_{100}$ und die in Gegenwart von Inhibitoren gemessene Extinktionsdifferenz mit $\Delta E$, so ergibt sich die Hemmung des Plasmins durch die Inhibitoren unter den angegebenen Bedingungen durch den folgenden Ausdruck

$$\frac{\Delta E_{100} - \Delta E}{\Delta E_{100}} \cdot 100\ \%$$

BAD ORIGINAL

Die in Tabelle 2 für einige BPTI-Derivate aufgeführten Hemmwerte (ausgedrückt in %) werden auf die durch die gleiche Menge BPTI bewirkte Hemmung (≙ 100 %) bezogen.

f) Trypsin-Inhibition

Die Bestimmung von Trypsin wurde mit Benzoyl-L-arginin-äthylester-hydrochlorid als Substrat im pH-Stat-Verfahren nach R.Ruyssen (Symposium on Pharmaceutical Enzymes and their Assay, Universitaire Pers, Ghent, Belgium 1969, S. 110) durchgeführt. Die in Tabelle 2 für einige BPTI-Derivate aufgeführte Hemmwerte wurden, wie unter Chymotrypsin-Inhibition angegeben, ermittelt.

**Tabelle 2** Hemmung einiger Serinproteasen durch BPTI-Derivate

| BPTI-Derivat aus Beispiel | Enzym: Chymotrypsin | Cathepsin G | Elastase (Pankr.) | Elastase (Gran.) | Kallikrein (Pankr.) | Kallikrein (Plasm.) | Plasmin | Trypsin |
|---|---|---|---|---|---|---|---|---|
| BPTI | 100 | 100 | 0 | 0 | 100 | 100 | 100 | 100 |
| 2 | 105 | 151 | 17 | 17 | 79 | 40 | 15 | 68 |
| 6 | 97 | 191 | 0 | 20 | 58 | 77 | 38 | 76 |
| 15 | 135 | 135 | 0 | 17 | 66 | 62 | 25 | 77 |
| 18 | 117 | 109 | 13 | 0 | 22 | 86 | 72 | 75 |
| 27 | 88 | 83 | 0 | 13 | 74 | 62 | 19 | 88 |
| 29 | 92 | 65 | 0 | 13 | 64 | 60 | 45 | 92 |
| 30 | 79 | 143 | 0 | 32 | 80 | 34 | 85 | 74 |
| 31 | 124 | 65 | 0 | 15 | 86 | 103 | 85 | 100 |
| 33 | 148 | 48 | 0 | 12 | 85 | 94 | 28 | 37 |
| 38 | 77 | 83 | 0 | 15 | 17 | 84 | 33 | 85 |
| 42 | 113 | 95 | 0 | 28 | 15 | 47 | 42 | 82 |
| 46 | 121 | 31 | 97 | 64 | 15 | 4 | 0 | 27 |
| 47 | 106 | 44 | 95 | 66 | 14 | 9 | 5 | 47 |
| 48 | 111 | 104 | 83 | 18 | 53 | 58 | 49 | 42 |

**Tabelle 2** Fortsetzung

| BPTI-Derivat aus Beispiel | Enzym<br>Chymo-trypsin | Cathep-sin G | Elastase (Pankr.) | Elastase (Gran.) | Kalli-krein (Pankr.) | Kalli-krein (Plasm.) | Plasmin | Trypsin |
|---|---|---|---|---|---|---|---|---|
| 50 | 123 | 226 | 2 | 19 | 15 | 66 | 24 | 38 |
| 54 | 116 | 40 | 67 | 15 | 11 | 54 | 5 | 15 |
| 56 | 104 | 78 | 14 | 0 | 8 | 104 | 25 | 13 |
| 57 | 130 | 113 | 71 | 9 | 35 | 61 | 15 | 35 |
| 62 | 147 | 80 | 0 | 0 | 7 | 72 | 29 | 28 |
| 65 | 59 | 195 | 0 | 17 | 8 | 23 | 33 | 51 |
| 67 | 78 | 160 | 26 | 26 | 8 | 25 | 24 | 26 |
| 68 | 57 | 208 | 15 | 26 | 12 | 36 | 29 | 75 |
| 72 | 74 | 217 | 25 | 49 | 41 | 61 | 81 | 68 |
| 75 | 45 | 0 | 0 | 28 | 90 | 66 | 55 | 43 |
| 77 | 97 | 58 | 10 | 39 | 22 | 28 | 15 | 19 |
| 78 | 97 | 115 | 0 | 36 | 43 | 45 | 32 | 30 |
| 79 | 156 | 99 | 21 | 26 | 35 | 59 | 19 | 21 |

Für Chymotrypsin, Cathepsin G, die Kallikreine, Plasma und Trypsin sind die angegebenen Hemmwerte bezogen auf die durch die gleiche Menge BPTI bewirkte Hemmung (100 %). Die BPTI-Menge im Hemmtest wurde so gewählt, daß die Absolutwerte der Hemmung 50 ± 10 % betrug, außer beim Cathepsin G, wo der Absolutwert bei 20 % lag.

Bei den Elastasen enthält die Tabelle Absolutwerte. Je Test wurden 25 µg BPTI-Derivat eingesetzt.

**Versuchsanordnung zum Nachweis entzündungshemmender Wirkung bei der Ratte**

a) Kaolin-induzierte Entzündungsreaktion

Die Entzündungsreaktion wurde durch intraplantare Injektion von 0,1 ml einer 10 %igen Kaolinsuspension (Bolus weiß, fein gepulvert, Merck AG, Darmstadt) in eine Hinterpfote von 130-160 g schweren Wistarratten induziert. Die für die Behandlung der Entzündungsreaktion verwendeten erfindungsgemässen BPTI-Derivate sowie BPTI wurden in 0,9 %iger Natriumchloridlösung in einer Konzentration von 10-20 mg/ml gelöst. Die Behandlung der Versuchstiere erfolgte durch intraperitoneale, intramuskuläre, subkutane oder intravenöse Injektion von 0,5 - 1,0 ml Lösung von BPTI-Derivaten und zum Vergleich BPTI entweder prophylaktisch, d.h. vor Setzen der Entzündungsnoxe, oder therapeutisch, d.h. nach Setzen der Entzündungsnoxe. Die Schwellung der entzündeten Pfote, die ein Maß für die Schwere der Entzündungsreaktion ist, wurde mit dem Antiphlogmeter nach Kemper /F.Kemper und G.Ameln, Z.ges.exp.Med. 131, 407-411 (1959)/ zeitlich verfolgt.

Zur Ermittlung der Dosis-Wirkungsbeziehungen wurde der 4 Stunden nach Setzen der Entzündungsnoxe gemessene Wert verwendet.

Der Wirkungsvergleich der erfindungsbemäßen BPTI-Derivate zeigt, daß sie dem BPTI in ihrer entzündungshemmenden Wirkung überlegen sind.

b) Aerosil-induzierte Entzündungsreaktion

Die Entzündungsreaktion wurde durch intraplantare Injektion von 0,1 ml einer 2 %igen Aerosilsuspension (Aerosil 200, Degussa AG, Frankfurt) in eine Hinterpfote von 130-160 g schweren Wistarratten induziert. Die für die Behandlung der Entzündungsreaktion verwendeten erfindungsgemässen BPTI-Derivate bzw. BPTI wurden in 0,9 %iger Natriumchloridlösung in einer Konzentration von 10-20 mg/ml gelöst. Die Behandlung der Versuchstiere erfolgt durch intraperitoneale, subkutane oder intravenöse Injektion von 0,5 - 1,0 ml Lösung von BPTI-Derivaten und zum Vergleich BPTI 15 Stunden nach Setzen der Entzündungsnoxe. Die Schwellung der entzündeten Pfote, die ein Maß für die Schwere der Entzündungsreaktion ist, wurde mit dem Antiphlogmeter nach Kemper zeitlich verfolgt. Zur Ermittlung der Dosis-Wirkungsbeziehungen wurde der 21-Stundenwert nach Entzündungsinduktion (= 6 Stunden nach Injektion der erfindungsgemäßen BPTI-Derivate bzw. des BPTI) ermittelt.

Das Ergebnis der Therapieversuche mit den erfindungsgemäßen BPTI-Derivaten zeigt die Wirksamkeit der verwendeten BPTI-Derivate in diesem experimentellen Modell, in dem BPTI in gleicher Dosierung die Entzündungsreaktion nicht hemmt.

Die erfindungsgemäßen neuen BPTI-Derivate sind dem BPTI als Arzneimittel überlegen. Von besonderem Vorteil sind ihre inhibitorischen Wirkungen auf die Elastasen aus Pankreas und Granulozyten, die neue therapeutische Einsatzmöglichkeiten eröffnen. Pankreas-Elastase spielt eine wichtige Rolle bei der Pankreatitis /M.C.Geokas, H.Rinderknecht, V.Swanson, B.P.Vitron und B.J. Haverback, Clin.Res. 16, 285 (1968)/; Serum-Elastase bei der Atherosklerose /U.Butturini und M.Langen, Klin.Wochenschr. 40, 472 (1962)/ und Granulozyten-Elastase bei akuten und

chronischen Entzündungen mit Bindegewebsschädigung /A.Janoff, Amer.J.Pathol. 68, 579 (1972)/, bei Gefäßwandschädigungen /A.Janoff und J.D. Zeligs, Science 161, 702 (1968)/, bei nekrotisierenden Erkrankungen und Degeneration von Lungengewebe, z.B. beim Emphysem / G.M.Turino, R.M.Senior, B.D.Garg, S.Keller, M.M.Levi und I.Mandl, Science 165, 709 (1969); H.E.Evans, M.M.Levi und I.Mandl, Amer.Rev.Respir.Dis. 101, 359 (1970) sowie A.Janoff, R.A.Sandhaus, V.D.Hospelhorn und R.Rosenberg, Proc.Soc.Exptl.Biol.Med. 140, 516 (1972)/. Ebenso wichtig ist die Rolle von lysosomalen Enzymen und insbesondere der Granulozyten-Elastase bei immunologisch bedingten Entzündungsreaktionen / M.Koono, M.Muto und H.Hayashi, Tohoku J.Exptl.Med. 94, 231 (1968) / , z.B. der rheumatoiden Arthritis / G.Weissmann und J. Spilberg, Arthritis Rheumat. 11, 162 (1968) /.

Es wurde gefunden, daß die erfindungsgemäßen BPTI-Derivate in Modellen der akuten Entzündungsreaktion dem BPTI überlegen sind, da mit ihnen nicht nur in deutlich geringeren Dosierungen die gleiche Wirkung wie mit BPTI erreicht wird, sondern die Entzündungsreaktion auch dann signifikant gehemmt wird, wenn sie mehrere Stunden nach Setzen der Entzündungsnoxe verabreicht werden. Eine solche therapeutische Wirkung ist mit dem BPTI im Kaolin- und Aerosilmodell bei einmaliger Gabe nicht zu erreichen.

Diese im Vergleich zu BPTI veränderte Wirkung und Wirksamkeit der erfindungsgemäßen BPTI-Derivate sind auf das veränderte Hemmspektrum und andere veränderte Eigenschaften, wie grössere Verweildauer und Wirkungszeit im Körper der Versuchstiere, zurückzuführen. Die erfindungsgemäßen BPTI-Derivate sind deshalb biologisch deutlich vom BPTI abzugrenzen.

Die neuen erfindungemäßen BPTI-Derivate des BPTI können aufgrund ihrer biologischen Wirksamkeit insbesondere zur Behandlung folgender Krankheiten bzw. Krankheitserscheinungen eingesetzt werden:

1. Verschiedene Formen des Schocks, posttraumatischer und postoperativer Komplikationen
2. Störungen der Blutgerinnung
3. Akute und chronische Entzündungsreaktionen, insbesondere zur Therapie und Prophylaxe von Organschädigungen, wie beispielsweise Pankreatitis und Strahlen-induzierte Enteritis
4. Immunkomplex-bedingte Entzündungsreaktionen, wie Immun-Vasculitis, Glomerulonephritis und Arthritiden
5. Kollagenosen, insbesondere rheumatoide Arthritis
6. durch Stoffwechsel-bedingte Ablagerungen verursachte Arthritiden (z.B. Gicht)
7. Degeneration der elastischen Bestandteile der Bindegewebsbestandteile von Organen wie bei der Atherosklerose und Lungenemphysem.

Die neuen Wirkstoffe können in bekannter Weise (analog BPTI) in die üblichen Formulierungen überführt werden.

Folgende Formulierungen sind dabei bevorzugt zu nennen:

1. Lösungen für parenterale Anwendung zur i.v.-, i.m.-, s.c.-Injektion, zur intraartikulären- und intratumoralen-Injektion
2. Lösungen für intravenöse Dauerinfusion
3. Lösungen zur Anwendung als Aerosole zur Inhalation
4. Lösungen, Emulsionen, Salben, Pasten, Cremes, Lotions, Puder zur äußerlichen lokalen Anwendung
5. Kombination verschiedener Hemmstoffe, deren Wirkungsspektrum sich gegenseitig ergänzt.

Die Konzentrationen der neuen Wirkstoffe in den erfindungsgemäßen Formulierungen bewegen sich in den Grenzen 0,01 bis 100 mg/ml Lösung, vorzugsweise zwischen 0,1 und 10 mg/ml Lösung.

Die neuen Wirkstoffe können in üblicher Weise angewendet werden, insbesondere sind folgende Anwendungsmethoden als bevorzugt zu nennen:

1. parenteral, intravenös, intramuskulär, subkutan, intraartikulär, intratumoral
2. lokal; als Aerosol
3. oral, evtl. in Magensaft-resistenten Anwendungsformen und/oder Dünndarm-löslich.

Als Dosierungsbereich kann für die neuen erfindungsgemäßen Verbindungen angegeben werden:

0,1 - 40 mg Wirkstoff / kg Körpergewicht, vorzugsweise 1 bis 25 mg Wirkstoff / kg Körpergewicht, die Dosierung ist dabei vor allem abhängig von der zu behandelnden Spezies sowie von der Applikationsart.

Die erfindungsgemäßen, neuen Wirkstoffe können bei Mensch und Tier eingesetzt werden.

Die im vorliegenden Anmeldungstext verwendeten Warenzeichen besitzen folgende Bedeutung:

Sephadex(R) G 10 = Quervernetztes Dextran mit einer Molgewichtsausschlußgrenze von 700

Sephadex(R) G 50 = Quervernetztes Dextran mit einer Molgewichtsausschlußgrenze von 30 000

SP-Sephadex(R) = Sulfopropylgruppen-haltiges quervernetztes Dextran

Synthese der für die Carboxyl-modifizierungen von BPTI und BPTI-Derivaten verwendeten nucleophilen Aminoverbindungen

$A_1$) Trans-4-aminomethyl-cyclohexan-1-carbonsäureamid-hydrobromid

a) Trans-4-carbobenzoxyaminomethyl-cyclohexan-1-carbonsäure

Die Lösung von 31.4 g Trans-4-aminomethyl-cyclohexan-1-carbonsäure (0.2 M) in 200 ml n Natronlauge wurde mit einer Lösung von 42.5 g Carbobenzoxychlorid (0.25 M) in 100 ml Aceton bei 40°C in 8 Anteilen versetzt. Der pH-Wert der Reaktionslösung wurde mit 4 n Natronlauge während der Umsetzung bei 9,6 gehalten. Dabei schied sich ein Niederschlag ab. Nach 4 Std. wurde die Suspension zweimal mit je 100 ml Äther extrahiert und durch Zugabe von 6 n Salzsäure ein pH-Wert von 1.0 eingestellt. Man extrahierte nun mit insgesamt 400 ml Äthylacetat in 3 Anteilen und engte die Auszüge nach dem Neutralwaschen i.Vak. ein. Der Rückstand wurde aus Acetonitril umkristallisiert. Man erhielt 35 g (48 %) farblose Kristalle vom Schmp. 116-118°C.

| | | | | |
|---|---|---|---|---|
| Ber. f. $C_{16}H_{21}NO_4$ (291.3) | | C 66.0 | H 7.3 | N 4.8 |
| | Gef. | 65.9 | 7.4 | 5.0 |

b) Trans-4-carbobenzoxyaminomethyl-cyclohexan-1-carbonsäureamid

27.1 g (0.1 Mol) Trans-4-carbobenzoxyaminomethyl-cyclohexan-1-carbonsäure wurden in 200 ml absol. Tetrahydrofuran gelöst. Nach dem Abkühlen der Lösung auf -10°C versetzte man diese unter Feuchtigkeitsausschluß mit 9.5 ml (0.1 Mol) Chlorameisensäure-äthylester und tropfte dann bei -5°C 11.2 ml N-Methylmorpholin (0.1 Mol) zu. 10 Min. nachdem das Eintropfen beendet war, versetzte man das Reaktionsgemisch mit 50 ml 10 n methanolischer Ammoniaklösung und rührte die Reaktionslösung 30 Min. bei 0°C und weitere 2 Std. bei 22°C. Dann wurde das ausgefallene

N-Methylmorpholin-hydrochlorid durch Abfiltrieren abgetrennt und das Filtrat i.Vak. eingeengt. Der Rückstand schmolz nach dem Umkristallisieren aus Acetonitril bei 186°C.

| | | | | |
|---|---|---|---|---|
| Ber. f. $C_{16}H_{22}N_2O_3$ (290.4) | | C 66.2 | H 7.6 | N 9.7 |
| | Gef. | 65.7 | 7.8 | 9.7 |

c) Trans-4-aminomethyl-cyclohexan-1-carbonsäureamid-hydrobromid

Aus 5.8 g der unter $A_1$b beschriebenen Carbobenzoxy-Verbindung wurden wie für das Nitril unter $A_2$b beschrieben durch Behandeln mit 15 ml einer 4 n Bromwasserstofflösung in Eisessig 4.5 g (95 %) des Amid-hydrobromids mit einem Schmp. von 245°C erhalten. $R_F$ SBA 0.43.

| | | | | | |
|---|---|---|---|---|---|
| Ber. f. $C_8H_{16}N_2O \cdot HBr$ (237.2) | | C 40.5 | H 7.2 | N 11.8 | Br 33.7 |
| | Gef. | 40.3 | 6.9 | 11.5 | 35.0 |

$A_2$) Trans-4-aminomethyl-1-cyano-cyclohexan-hydrobromid

a) Trans-4-carbobenzoxyaminomethyl-1-cyano-cyclohexan

29 g des nach $A_1$b) erhaltenen Amids (0,1 Mol) wurden in 80 ml absol. Pyridin gelöst, und die Lösung wurde bei 50°C mit 16 ml Benzolsulfonsäurechlorid versetzt. Man erhitzte die Reaktionsmischung 30 min. auf 55°C und fügte nach dem Abkühlen auf 20°C 200 g Eis zu der Mischung und konz. Salzsäure bis zum Erreichen von pH 1.5. Man isolierte die ausgefallene Substanz durch Abfiltrieren und wusch den Niederschlag mit Eiswasser bis zum negativen Ausfallen des Chloridnachweises im Filtrat. Nach dem Trocknen und Umkristallisieren des Filterkuchens aus Äthylacetat-Petroläther und schließlich aus Äthylacetat wurden 20.1 g (72 %) farblose Kristalle vom Schmp. 116-117°C erhalten.

BAD ORIGINAL

Ber. f. $C_{16}H_{20}N_2O_2$ (272.3) C 70.6 H 7.4 N 10.3
Gef. 70.3 7.5 9.9

b) Trans-4-aminomethyl-1-cyano-cyclohexan-hydrobromid

Zu einer unter gelindem Erwärmen erhaltenen Lösung von 5.4 g (20 m Mol) des nach $A_2a$) erhaltenen Carbobenzoxyderivats in 10 ml Eisessig fügte man unter Feuchtigkeitsausschluß 15 ml einer 4 n Bromwasserstofflösung in Eisessig. Nach 1-stündigem Stehen bei 25°C vervollständigte man die Fällung des Hydrobromids durch Zugabe von 100 ml absol. Äthylacetat und isolierte den Niederschlag durch Abfiltrieren. Nach dem Umfällen aus 60 ml Chloroform-Acetonitril (1:1) und absol. Äther wurden 3.6 g (62 %) farblose, bei 212-215°C schmelzende Kristalle erhalten. $R_F$ SBA*) 0.57.

Ber. f. $C_8H_{14}N_2 \cdot HBr$ (219.1) C 43.8 H 6.9 N 12.8 Br 36.5
Gef. 42.0 6.9 12.6 37.8

$A_3$) Trans-4-aminomethyl-cyclohexan-1-carboxyamidin-dihydrobromid

a) Trans-4-carbobenzoxyaminomethyl-cyclohexan-1-carboxamidin-hydrochlorid

5.4 g Trans-4-carbobenzoxyaminomethyl-1-cyano-cyclohexan (20 m Mol) (vgl. $A_2a$)) wurden unter Erwärmen in einem Gemisch aus 0.8 ml Methanol und 5 ml Toluol gelöst. In die nach dem Abkühlen auf 0°C erhaltene Suspension leitete man bis zur vollständigen Auflösung Chlorwasserstoff ein und hielt die Mischung 48 Std. bei -60°C. Die Lösung wurde i.Vak. zur Trockne eingedampft und der sirupöse Rückstand in 20 ml gesättigtem methanolischem Ammoniak gelöst. Diese Lösung wurde nach 4 Std. Stehen bei 25°C eingeengt und die äthanolische

---

*) SBA: sek. Butanol : Ameisensäure : Wasser =75:10:15 (Vol)

Lösung des sirupösen Rückstandes in 150 ml absol. Äther eingetropft. Im Exsiccator erstarrte das sirupöse Präzipitat zu einem farblosen festen Schaum. Ausbeute 2.65 g (40 %).

| | | | | | |
|---|---|---|---|---|---|
| Ber. f. $C_{16}H_{23}N_3O_2 \cdot HCl$ (325.8) | | C 60.0 | H 7.4 | N 12.9 | Cl 10.9 |
| | Gef. | 58.4 | 6.9 | 12.9 | 11.4 |

b) Trans-4-aminomethyl-cyclohexan-1-carboxamidin-dihydrobromid

2.6 g des unter $A_3$a) beschriebenen Carbobenzoxy-Derivates wurden, wie für das unter $A_2$b) beschriebene Nitril angegeben, mit 5 ml einer 4 n Bromwasserstofflösung in Eisessig umgesetzt. Nach 1-stündiger Reaktion erhielt man durch Fällen mit absol. Äther 2.5 g einer sehr hygroskopischen, bei 242°C unter Zers. schmelzenden farblosen Substanz (98 % Ausbeute).

| | | | | | |
|---|---|---|---|---|---|
| Ber. f. $C_8H_{17}N_3 \cdot 2HBr$ (317.1) | | C 30.3 | H 6.0 | N 13.3 | Br 50.4 |
| | Gef. | 30.4 | 5.8 | 12.3 | 48.8 |

$A_4$) ε-tert.-Butoyloxycarbonyl-L-lysin-amid

a) α-Carbobenzoxy-ε-tert.-butyloxycarbonyl-L-lysin-amid

17 g (30 m Mol) α-Carbobenzoxy-ε-tert.butyloxycarbonyl-L-lysin-dicyclohexylammoniumsalz /E.Schnabel, Liebigs Ann. Chem. 674, 218 (1964)/ wurden in 130 ml absol. Dimethylformamid, wie bei der unter $A_1$b beschriebenen Synthese von Trans-4-carbobenzoxyaminomethyl-cyclohexan-1-carbonsäureamid mit Chlorameisensäureäthylester zum gemischten Anhydrid umgesetzt und dieses in das entsprechende Amid übergeführt. Ausbeute nach dem Umkristallisieren aus Acetonitril: 9.4 g (82 %); Schmp.: 142-143°C;

$/\alpha/_{578}^{20}$ = +3.6° (c = 1, in Dimethylformamid).

BAD ORIGINAL

b) ε-tert.-Butyloxycarbonyl-L-lysin-amid

9 g (24 m Mol) der unter a) beschriebenen Carbobenzoxy-Verbindung wurden in 100 ml Äthanol gelöst. Zu der Lösung fügte man aus 3.5 g Palladiumchlorid frisch bereiteten Palladium-mohr und ließ unter gutem Durchmischen einen langsamen Wasserstoffstrom durch die Suspension perlen. Nach 5 Std. wurde der Katalysator durch Abfiltrieren abgetrennt. Nach dem Abdestillieren des Alkohols i.Vak. blieben farblose Kristalle zurück. Sie schmolzen nach dem Umkristallisieren aus Acetonitril und aus Äthylacetat-Petroläther bei 99-100°C; $R_F$ SBA 0.42; Ausbeute 5 g (86 %);
$/\alpha/_{578}^{20} = + 15.4°$ (c = 1, in Eisessig).

| | | C | H | N |
|---|---|---|---|---|
| Ber. f. $C_{11}H_{23}N_3O_3$ (245.3) | | 53.8 | 9.5 | 17.1 |
| | Gef. | 53.4 | 9.5 | 17.2 |

A5) L-Ornithinamid-dihydrochlorid

a) $N^\alpha,N^\delta$-Bis-tert.-butyloxycarbonyl-L-ornithin

26 g L-Ornithin (0.2 Mol) wurden nach dem üblichen Verfahren /E.Schnabel, Liebigs Ann.Chem. 702, 188 (1967)/ bei pH 9.5 mit tert.Butyloxycarbonylazid acyliert. Durch Umsetzung der zunächst sirupösen Substanz mit Dicyclohexylamin wurde das kristallisierte Dicyclohexylammoniumsalz in 64 %iger Ausbeute erhalten = 65.2 g vom Schmp. 166°C.

| | | C | H | N |
|---|---|---|---|---|
| Ber. f. $C_{15}H_{25}N_2O_6 \cdot C_{12}H_{23}N$ (513.7) | | 63.1 | 10.0 | 8.2 |
| | Gef. | 63.1 | 9.9 | 8.2 |

Das $N\alpha,N\delta$-Bis-tert.butyloxycarbonyl-L-ornithin kristallisierte nach dem Zerlegen des Dicyclohexylammoniumsalzes mit 2 n Schwefelsäure beim Stehen unter Diisopropyläther und schmolz bei 112-113°C; $[\alpha]_{578}^{21}$ = - 5.9° (c = 1.1, in Eisessig).

| | | | |
|---|---|---|---|
| Ber. f. $C_{15}H_{28}N_2O_6$ (332.4) | C 54.2 | H 8.5 | N 8.4 |
| Gef. | 54.2 | 8.6 | 8.4 |

b) <u>$N\alpha,N\delta$-Bis-tert.butyloxycarbonyl-L-ornithinamid</u>

Aus 26 g (78 m Mol) des unter a) beschriebenen Ornithinderivates wurden analog zu $A_1b$ 22 g (55 %) des Amids erhalten, die nach dem Umkristallisieren aus Acetonitril bei 153-154°C schmolzen. $[\alpha]_{578}^{25}$ = - 0.5° (c = 1, in Eisessig).

| | | | |
|---|---|---|---|
| Ber. f. $C_{15}H_{29}N_3O_5$ (331.4) | C 54.3 | H 8.8 | N 12.7 |
| Gef. | 55.0 | 9.1 | 12.7 |

c) <u>L-Ornithinamid-dihydrochlorid</u>

11 g (33 m Mol) des unter b) beschriebenen Amids wurden in 20 ml Trifluoressigsäure gelöst. Nach 30 Min. Stehen bei 22°C versetzte man die Lösung mit 50 ml einer 6 n ätherischen Chlorwasserstofflösung. Das ausgefällte Dihydrochlorid wurde durch Abfiltrieren isoliert. Man erhielt 6.5 g (96 %) farblose Kristalle mit einem Schmp. von 196°C (Zers.); $[\alpha]_{578}^{22}$ = + 15.3° (c = 1, in Trifluoressigsäure).

| | | | | |
|---|---|---|---|---|
| Ber. f. $C_5H_{13}N_3O\cdot 2HCl$ (204.1) | C 29.4 | H 7.4 | N 20.6 | Cl 34.7 |
| Gef. | 29.6 | 7.5 | 20.4 | 34.5 |

$A_6$) N-Methyl-L-threoninol

Die Lösung von 27 g Carbobenzoxy-L-threonin-methylester (0.1 Mol) /C.A.Dekker, S.P.Taylor und J.S.Fruton, J.Biol.Chem. 180, 155 (1949)/ in 300 ml absol. Tetrahydrofuran wurde unter Rühren und Kühlen tropfenweise mit einer Lösung von 12 g Lithiumalanat (0.31 Mol) in Tetrahydrofuran versetzt. Nach dem Zutropfen wurde das Kühlbad entfernt und die Reaktionslösung 1 Std. am Rückflußkühler zum Sieden erhitzt. Die abgekühlte Reaktionsmischung versetzte man vorsichtig mit 50 ml Eiswasser und saugte die ausgefallenen Hydroxide ab. Den Rückstand extrahierte man mehrfach mit Methanol. Die vereinigten Filtrate wurden eingeengt und der sirupartige Rückstand fraktioniert destilliert. Bei $Kp_{0.5}102^o$ destillierten 8.9 g (75 %) einer farblosen, zähen Flüssigkeit über.

$/\alpha/^{22}_{578} = - 27.4^o$ (c = 1, in Dimethylformamid).

| | | | | |
|---|---|---|---|---|
| Ber. f. $C_5H_{13}NO_2$ (119.2) | | C 50.4 | H 11.0 | N 11.8 |
| | Gef. | 49.9 | 10.7 | 11.9 |

$A_7$) $\gamma$-tert.-Butyl-L-glutamyl-L-glutaminsäure-di-tert.-butylester

a) Carbobenzoxy-$\gamma$-tert.-butyl-L-glutamyl-L-glutaminsäure-di-tert.-butylester

24 g Carbobenzoxy-L-glutaminsäure-$\gamma$-tert.butylester (70 m Mol) /R.Schwyzer und H.Kappeler, Helv.Chim.Acta 44, 1991 (1961)/ und 18.5 g L-Glutaminsäure-di-tert.butylester (70 m Mol) /G.W.Anderson und F.M.Callahan, J.Amer. Chem.Soc. 82, 3359 (1966)/ wurden in 100 ml absol. Tetrahydrofuran gelöst. Nach dem Abkühlen der Lösung auf -5°C trug man 14.6 g festes Dicyclohexylcarbodiimid ein und hielt

das Gemisch 2 Tage bei +4°C . Dann wurde der ausgefallene Dicyclohexylharnstoff durch Absaugen abgetrennt und das Filtrat i.Vak. eingeengt. Man löste den sirupösen Rückstand in 250 ml Äthylacetat und extrahierte die Lösung nacheinander mit 10 %iger Zitronensäurelösung, mit 5 %iger wässriger Natriumbicarbonatlösung und schließlich mit Wasser. Das Äthylacetat wurde i.Vak. abdestilliert. Der zurückgebliebene Sirup kristallisierte beim Stehen unter wenig Petroläther. Man erhielt 31 g farblose Kristalle mit Schmp. 84-86°C; $R_F$ CMA*) 0.83; $/\alpha/_{578}^{24}$ = - 18.2° (c = 1.05, in Dimethylformamid).

| | | | | |
|---|---|---|---|---|
| Ber. f. $C_{30}H_{46}N_2O_9$ (578.7) | | C 62.3 | H 8.1 | N 4.8 |
| | | Gef. 62.6 | 8.3 | 4.6 |

b) γ-tert.Butyl-L-glutamyl-L-glutaminsäure-di-tert.butylester

29 g der unter A 7a beschriebenen Carbobenzoxyverbindung (50 m Mol) wurden in 150 ml absol. Äthanol gelöst. Durch diese Lösung leitete man nach Zusatz von ca. 1 g frisch dargestelltem Palladium-Mohr unter gutem Durchmischen mit einem Vibromischer einen langsamen Wasserstoffstrom. Nach 12 Std. wurde der Katalysator abfiltriert und das Filtrat i.Vak. eingeengt. Es hinterblieben 19.1 g (86 %) eines viskosen Öles.
$R_F$ SBA 0.78; $/\alpha/_{578}^{22}$ = - 17.1° (c = 1, in Dimethylformamid).

| | | | | |
|---|---|---|---|---|
| Ber. f. $C_{22}H_{40}N_2O_7$ (444.6) | | C 59.5 | H 9.1 | N 6.3 |
| | | Gef. 59.9 | 9.3 | 6.2 |

---

*) CMA: Chloroform : Methanol : Essigsäure = 190 : 10 : 6 (Vol.)

$A_8$) Di-(γ-tert.-butyl-L-glutamyl)-glutaminsäure-di-tert.-butylester

a) Carbobenzoxyderivat

Aus 13.5 g Carbobenzoxy-L-glutaminsäure-γ-tert.-butylester (40 m Mol) und 15 g γ-tert.Butyl-L-glutamyl-glutaminsäure-di-tert.-butylester (34 m Mol) gewann man analog zu $A_7$a durch Carbodiimid-Kondensation in 150 ml absol. Dimethylformamid 20.5 g farblose Kristalle (87 %). Die Substanz schmolz bei 110°C, der $R_F$ CMA lag bei 0.89.
$/\alpha/_{578}^{24}$ = - 17.9° (c = 1, in Dimethylformamid).

| | | | | |
|---|---|---|---|---|
| Ber. f. $C_{39}H_{61}N_3O_{12}$ (763.9) | | C 61.3 | H 8.0 | N 5.5 |
| | Gef. | 61.1 | 8.0 | 5.5 |

b) katalytische Hydrierung

13.5 g des unter $A_8$a beschriebenen Carbobenzoxyderivates wurden in 200 ml absol. Äther, wie für $A_7$b angegeben, hydriert. Nach dem Abdestillieren des Äthers wurden 10.1 g (91 %) farbloser Sirup erhalten. $R_F$ SBA 0.70; $R_F$ SBN *) 0.92; $/\alpha/_{578}^{24}$ = - 16.6° (c = 1, in Dimethylformamid).

| | | | | |
|---|---|---|---|---|
| Ber. f. $C_{31}H_{55}N_3O_{10}$ (629.8) | | C 59.1 | H 8.8 | N 6.7 |
| | Gef. | 59.5 | 8.9 | 6.5 |

$A_9$) L-Asparagyl-α,β-di-(L-glutaminsäure-di-tert.-butylester)

a) Carboxybenzoxy-L-asparagyl-α,β-di-(L-glutaminsäure-di-tert.-butylester)

7 g Carbobenzoxy-asparaginsäure (26 m Mol) /M.Bergmann und L.Zervas, Ber.dt.chem.Ges. 65, 1192 (1932)/ und 13.6 g

*) SBN: sek. Butanol : 10 % ig.Ammoniak = 85 : 15 (Vol.)

L-Glutaminsäure-di-tert.butylester (55 m Mol) /G.W.Anderson und F.M.Callahan, J.Amer.Chem.Soc. 82, 3359 (1960)/ wurden unter Zusatz von 7.5 g N-Hydroxybenztriazol in 150 ml absol. Dimethylformamid gelöst und die Lösung nach dem Abkühlen auf -5°C mit 11.3 g Dicyclohexylcarbodiimid (55 m Mol versetzt. Das Reaktionsgemisch wurde nach 2-tägigem Stehen bei +4°C aufgearbeitet, wie bei $A_7a$ beschrieben. Beim Versetzen der ätherischen Lösung des öligen Eindampfrückstandes mit Petroläther wurden 12.3 g (63 %) einer farblosen amorphen Substanz erhalten. Schmp. 90°C; $R_F$ CMA 0.88; $/\alpha/_{578}^{22}$ = - 15.9° (c = 1, in Dimethylformamid).

| | | | | |
|---|---|---|---|---|
| Ber. f. $C_{38}H_{59}N_3O_{12}$ (749.9) | | C 60.9 | H 7.9 | N 5.6 |
| | Gef. | 60.7 | 7.8 | 5.6 |

b) katalytische Hydrierung

Aus 8.5 g der unter $A_9a$ beschriebenen Carbobenzoxyverbindung wurden analog zu $A_7b$ durch katalytische Hydrierung in 250 ml absol. Äther 6.5 g farblose, sirupartige Substanz erhalten (93 %).
$R_F$ SBA 0.75; $R_F$ SBN 0.92;
$/\alpha/_{578}^{23}$ = - 23.5° (c = 1, in Dimethylformamid).

| | | | | |
|---|---|---|---|---|
| Ber. f. $C_{30}H_{53}N_3O_{10}$ (615.8) | | C 58.5 | H 8.7 | N 6.8 |
| | Gef. | 59.3 | 8.3 | 6.7 |

$A_{10}$) L-Asparagyl-α,β-di-(L-serin-tert.-butyläther-tert.-butylester)

a) Carbobenzoxy-L-asparagyl-α,β-di-(L-serin-tert.-butyläther-tert.-butylester

7 g Carbobenzoxy-asparaginsäure und 12 g L-Serin-tert.-butyl-

äther-tert.butylester (55 m Mol) wurden analog zu $A_9a$ mit Hilfe von Dicylclohexylcarbodiimid und N-Hydroxybenztriazol kondensiert. Das Peptidderivat fiel nach dem Aufarbeiten gemäß $A_9a$ sirupös und chromatographisch uneinheitlich an. Man löste den Sirup in ca. 50 ml absol. Äther und trug die Lösung auf eine Säule mit Kieselgel 60 (2.5 x 12 cm) auf und eluierte diese nacheinander mit Petroläther, Petroläther-Äther (1:1) und Äther. Nach dem Abdestillieren des Petroläthers aus dem Petroläther-Eluat wurden 15.3 g farbloses, hochviskoses Öl mit $R_F$ SBA 0.92 chromatographisch rein erhalten (89 %); $/\alpha/_{578}^{23}$ = + 9.6° (c = 1, in Dimethylformamid).

| | | | | |
|---|---|---|---|---|
| Ber. f. $C_{34}H_{55}N_3O_{10}$ (665.8) | | C 61.3 | H 8.3 | N 6.3 |
| | Gef. | 61.6 | 8.2 | 6.4 |

15b) katalytische Hydrierung

Aus 10.5 g der unter $A_{10}a$ beschriebenen Carbobenzoxyverbindung wurden durch katalytische Hydrierung analog zu $A_8b$ eine zunächst sirupöse, chromatographisch nicht einheitliche Substanz erhalten. Durch Chromatographie an Kieselgel 60 wurden analog zu $A_{10}a$ mit Petroläther 5.7 g (68 %) Substanz isoliert, die unter Äther-Petroläther kristallisierte. Schmp. 70°C; $R_F$ SBA 0.65; $R_F$ SBN 0.92; $R_F$ CMA 0.12; $/\alpha/_{578}^{23}$ = + 3.9° (c = 1, in Dimethylformamid).

| | | | | |
|---|---|---|---|---|
| Ber. f. $C_{26}H_{49}N_3O_8$ (531.7) | | C 58.7 | H 9.3 | N 7.9 |
| | Gef. | 58.2 | 9.3 | 7.8 |

## Umsetzung von BPTI und BPTI-Derivaten mit nukleophilen Aminoverbindungen und Carbodiimiden

### Beispiel 1

Zu der Lösung von 1 g BPTI (154 µ Mol) in 100 ml Wasser fügte man 2.7 g Ammoniumchlorid (50 m Mol) und nach dem Einstellen auf einen pH-Wert von 4.75 1.95 g N-Äthyl-N'-(3-dimethylaminopropyl)-carbodiimid-hydrochlorid (10 m Mol). Durch Zugabe von n-Salzsäure wurde der eingestellte pH-Wert mit Hilfe eines Autotitrators aufrechterhalten. Nach 3 Std. fügte man 1.7 g Natriumacetat zu der Lösung und engte die Lösung durch Ultrafiltration mit einer UM 05-Diaflow-Ultrafiltrationsmembrane auf ein Volumen von ca. 10 ml ein. Das Retentat wurde durch Filtrieren über Sephadex G 10 entsalzt und von niedrigmolekularen Anteilen befreit. Die höhermolekularen Begleitstoffe - 75 mg (7.5 %) - wurden durch Chromatographie an Sephadex$^{(R)}$ G 50 mit 0,05 m Ammoniumacetatlösung abgetrennt. Nach zweimaliger Gefriertrocknung der Monomerfraktion wurden 720 mg farblose Substanz (72 %) erhalten. Relative elektrophoretische Beweglichkeit: - 1.44 (Cellogel) bzw. 1.08 (Diskelektrophorese).

$/\alpha/_{578}^{22}$ = 107.7° (c = 0.5, in Wasser).

### Beispiel 2

Zu einer Lösung von 1.3 g BPTI (200 µ Mol) und 2.4 g N-Methyl-L-threoninol (20 m Mol) in 130 ml Wasser fügte man bei 0°C 1.9 g N-Äthyl-N'-(3-dimethylaminopropyl)-carbodiimid-hydrochlorid (10 m Mol) und hielt die Reaktionslösung 6 Std. unter Rühren bei 22°C unter Einhaltung eines pH-Wertes von 4.85. Dann wurde, wie in Beispiel 1 beschrieben, aufgearbeitet. Neben 130 mg polymeren Anteilen (10 %) isolierte man 1.1 g farblose Substanz (85 %). Relative elektrophoretische Beweglichkeit:-1.26 (Cellogel) bzw. - 1.14 (Diskelektrophorese).

$/\alpha/_{578}^{22}$ = - 109° (c = 0.5, in Wasser).

Beispiel 3

Aus 1.3 g BPTI (200 μ Mol) und 1.5 g Neobornylamin (10 m Mol) erhielt man nach Beispiel 2 mit Hilfe von 1.45 g N-Äthyl-N'-(3-dimethylaminopropyl)-carbodiimid-hydrochlorid ein Kondensationsprodukt. Ausbeute: 1120 mg (86 %) sowie 92 mg polymere Anteile (7 %).
Relative elektrophoretische Beweglichkeiten: - 1.2 bzw. - 1.65 (2 Banden; Cellogel).

Beispiel 4

1 g BPTI (154 μ Mol) und 1.47 g Taurin (10 m Mol) wurden in 100 ml Wasser gelöst. Zu der Lösung gab man nach dem Einstellen eines pH-Wertes von 4.75 2.1 g N-Cyclohexyl-N'-(2-morpholino-äthyl)-carbodiimid-metho-p-toluolsulfonat (5 m Mol) und arbeitete die Reaktionslösung nach 5 Std. auf, wie für Beispiel 2 beschrieben. Durch Chromatographie an Sephadex G 50 wurden 925 mg (92 %) farblose Substanz erhalten.
Relative elektrophoretische Beweglichkeit: - 1.21 (Cellogel) bzw. 1.06 (Diskelektrophorese);
$/\alpha/_{578}^{20} = -73.2^{\circ}$ (c = 0.5, in Wasser).

Beispiel 5

Zu einer Lösung von 1.3 g BPTI (200 μMol) und 7.15 g Glycinamidhydrochlorid (6.5 mMol) /H.E.Johnson u. D.G.Crosby, J.Org.Chem. 27, 798 (1962)/ in 130 ml Wasser fügte man nach dem Einstellen eines pH-Wertes von 4.75 2.5 g N-Äthyl-N'-(3-dimethylaminopropyl) carbodiimid-hydrochlorid (1.3 mMol) und hielt die Reaktionsmischung unter Rühren 6 Std. bei Raumtemperatur. Man dialysierte den Ansatz in einem acetylierten Dialyseschlauch (L.C.Craig und

T.P.King in Methods of Biochemical Analysis Vol. X, S.175; Hrsg.: D.Glick, Interscience publishers New York/London 1962) gegen Wasser und lyophilisierte das Retentat. Zum Abtrennen der Salze filtrierte man das Lyophilisat über Sephadex(R) G 10 und trennt die höherpolymeren Anteile (55 mg = 4 %) durch Chromatographie an Sephadex G 50 mit 0.1 m Essigsäure als Elutionsmittel ab. Die Ausbeute an farbloser Substanz betrug 1050 mg (80 %).
Relative elektrophoretische Beweglichkeit: - 1.48 (Cellogel) bzw. - 1.15 (Diskelektrophorese);
Quantitave Aminosäureanalyse: 9.6 Gly/Mol (BPTI : 6);
$/\alpha/_{578}^{20}$ = - 100.2° (c = 0.5, in Wasser).

Beispiel 6

Nach dem in Beispiel 5 beschriebenen Verfahren wurden aus 1.3 g BPTI (200 µMol) und 3.4 g L-Glutaminamid-hydrobromid (15 mMol) /C.A.Dekker, D.Stone und J.S.Fruton, J.Biol.Chem. 181, 719 (1949)/ mit Hilfe von 1.92 g N-Äthyl-N'-(3-dimethylaminopropyl)-carbodiimid-hydrochlorid (10 mMol) 908 mg (70 %) Kondensationsprodukt erhalten.
Relative elektrophoretische Beweglichkeit: - 1.34 (Cellogel) bzw. - 1.12 (Diskelektrophorese);
Quantitative Aminosäureanalyse: 7.5 Glu/Mol (BPTI : 3);
$/\alpha/_{578}^{22}$ = - 103.1° (c = 0.5, in Wasser).

Beispiel 7

2.6 g BPTI (400 µMol) und 10.4 g L-Glutaminsäure-di-tert.butylester (40 mMol) /G.W.Anderson und F.M.Callahan, J.Amer.Chem.Soc. 82, 3359 (1960)/ wurden in 200 ml Wasser gelöst und bei pH-Wert 5.0 mit 3.85 g N-Äthyl-N'-(3-dimethylaminopropyl)-carbodiimid-hydrochlorid (20 mMol), wie in Beispiel 5 beschrieben, umgesetzt. Die Reaktionslösung wurde analog aufgearbeitet. Man erhielt nach Chromatographie an Sephadex(R) G 50 110 mg (4 %) polymere Anteile und 2032 mg (78 %) monomere Substanz.
Relative elektrophoretische Beweglichkeit: - 1.45 (Cellogel) bzw. - 1.02 (Diskelektrophorese); Aminosäureanalyse: 6.6 Glu/Mol) (BPTI : 3);

$/\alpha/_{578}^{20}$ = - 104.9° (c = 0.5, in Wasser).

Beispiel 8

Die Lösung von 785 mg BPTI-Derivat nach Beispiel 7 in 7 ml wasserfreier Trifluoressigsäure wurde 1 Std. bei Raumtemperatur unter Stickstoffatmosphäre gehalten. Dann setzte man 20 ml absol. Äther zu der Lösung und wusch den erhaltenen Niederschlag nach dem Absaugen mit Äther, Pyridin-Äther (1:4) und schließlich mit Äthylacetat. Den Rückstand löste man in Wasser und entsalzte die Lösung durch Filtrieren über Sephadex(R) G 10 mit 0.1 m Essigsäure als Elutionsmittel. Durch Gefriertrocknen der Protein enthaltenden Filtrate isolierte man 780 mg (~100 %) farblose Substanz.
Relative elektrophoretische Beweglichkeit: - 0.96 (Cellogel) bzw. - 1.06 (Diskelektrophorese); Aminosäureanalyse: 6.3 Glu/Mol (BPTI : 3).

Beispiel 9

1.3 g BPTI (200 µMol) wurden, wie in Beispiel 7 angegeben, mit L-Glutaminsäure-di-tert.butylester umgesetzt, wobei man anstelle von Wasser 100 ml 90 %iges Dimethylsulfoxid als

Lösungsmittel verwendete. Nach beendigter Umsetzung wurde das Lösungsmittelgemisch im Hochvakuum abdestilliert und der Rückstand mehrmals mit Äther gewaschen. Man löste das erhaltene Produkt in 10 ml absol. Trifluoressigsäure, engte die Lösung auf 2 ml ein und filtrierte den Rückstand nach dem Lösen in 10 ml Wasser über Sephadex G 10 mit 1 m Essigsäure. Die Protein enthaltenden Fraktionen wurden nach dem Gefriertrocknen an Sephadex G 50 mit 0.1-m Essigsäure als Laufmittel chromatographiert. Man isolierte schliesslich 850 mg farblose Substanz. Relative elektrophoretische Beweglichkeit: - 0.8 (Cellogel) bzw. - 1.0 (Diskelektrophorese); Aminosäureanalyse: 4,75 Glu/Mol (BPTI : 3).

Beispiel 10

Aus 1.3 g BPTI (200 µMol) und 4 g L-Phenylalanin-tert.butylester (18 mMol) /G.W.Anderson und F.M.Callahan, J.Amer.Chem.Soc. 82 3359 (1960)/ wurde nach der in Beispiel 7 angegebenen Methode mit Hilfe von 1.92 g (10 mMol) N-Äthyl-N'-(3-dimethylaminopropyl)-carbodiimid-hydrochlorid bei pH-Wert 4.9 ein Kondensationsprodukt erhalten, das nach Chromatographie an Sephadex G 50 56 mg polymere Anteile (4 %) und 1020 mg monomere Substanz (78 %) enthielt. Relative elektrophoretische Beweglichkeit: - 1.4 (Cellogel) bzw. - 1.11 (Diskelektrophorese); Aminosäureanalyse: 5.95 Phe/Mol (BPTI : 4);

$/\alpha/_{578}^{22}$ = - 97.0° (c = 0.5, in Wasser).

Beispiel 11

Die Lösung von 820 mg des nach Beispiel 10 erhaltenen BPTI-Derivates (125 µMol) in 10 ml wasserfreier Trifluoressigsäure wurde 1 Std. unter Stickstoffatmosphäre gehalten. Man versetzte die Lösung dann mit 30 ml absol. Äther und isolierte die ausgefallene Substanz durch Zentrifugation. Man wusch das Sediment durch mehrmaliges Aufschlämmen mit Äther unter Zusatz von Pyridin und schließlich mit Äthylacetat und chromatographierte die mit 0.1 m Essigsäure erhaltene Lösung über Sephadex(R) G 10. Beim Gefriertrocknen der Protein enthaltenden Filtrate wurden 810 mg (~100 %) farblose Substanz erhalten.
Relative elektrophoretische Beweglichkeit: - 1.25 und - 1.5 (Doppelbande; Cellogel) bzw. - 1.12 (Diskelektrophorese);
Aminosäureanalyse: 6.23 Phe/Mol (BPTI : 4).

Beispiel 12

1.3 g BPTI (200 µMol) und 4.35 g L-Phenylalaninamid (22 mMol) /K.Blau und S.G.Waley, Biochem.J. 57, 538 (1954)/ wurden, wie in Beispiel 8 beschrieben, in 130 ml Wasser mit 2.4 g N-Äthyl-N'-(3-dimethylaminopropyl)-carbodiimid-hydrochlorid (12.5 mMol) umgesetzt. Das Reaktionsgemisch wurde entsprechend aufgearbeitet. Man erhielt neben 105 mg polymeren Anteilen (8 %) 940 mg farblose Substanz (72 %).
Relative elektrophoretische Beweglichkeit: - 1.6 (Cellogel) bzw. 1.05 (Diskelektrophorese); Aminosäureanalyse: 6.3 Phe/Mol (BPTI : 4);
$/\alpha/_{578}^{20}$ = -108.5° (c = 0.5, in Wasser).

Beispiel 13

Aus 2.6 g BPTI (400 µMol) und 4.65 g D-Phenylalanin-tert.butylester (21 mMol) /G.W.Anderson und F.M.Callahan, J.Amer.Chem.Soc. 82, 3359 (1960)/ stellte man mit Hilfe von 2.9 g N-Äthyl-N'-(3-dimethylaminopropyl)-carbodiimid-hydrochlorid (15 mMol), wie in Beispiel 5 beschrieben, ein Kondensationsprodukt her. Man erhielt 1.95 g farblose Substanz (75 %). Relative elektrophoretische Beweglichkeit: - 1.36 (Cellogel) bzw. - 1.10 (Diskelektrophorese); Aminosäureanalyse: 5.93 Phe/Mol (BPTI : 4);

$/\alpha/^{22}_{578}$ = - 104.5° (c = 0.5, in Wasser).

Beispiel 14

Aus 750 mg (115 µMol) BPTI-Derivat nach Beispiel 13 wurden durch Lösen in 7 ml wasserfreier Trifluoressigsäure und halbstündiges Aufbewahren unter Stickstoff nach dem für Beispiel 14 angegebenen Verfahren 633 mg (84 %) farblose Substanz erhalten. Relative elektrophoretische Beweglichkeit: - 1.08 (Cellogel) bzw. - 1.05 (Diskelektrophorese); Aminosäureanalyse: 6.36 Phe/Mol (BPTI : 4);

$/\alpha/^{20}_{578}$ = - 98.9° (c = 0.5, in Wasser).

Beispiel 15

Durch Kondensation von 1.3 g BPTI (200 µMol) und 1.51 g D-Phenylalaninamid-hydrochlorid (7.5 mMol) - dargestellt analog dem L-Phenylalaninderivat aus Beispiel 12 - mit Hilfe von 1.44 g N-Äthyl-N'-(3-dimethylaminopropyl)-carbodiimid-hydrochlorid (7.5 mMol) bei einem pH-Wert von 5.0 wurden, wie in Beispiel 5 beschrieben, 1120 mg monomeres Kondensationsprodukt erhalten (86 %

Relative elektrophoretische Beweglichkeit: - 1.45 (Cellogel) bzw. - 1.08 (Diskelektrophorese); Aminosäureanalyse: 7.13 Phe/Mol (BPTI : 4).

Beispiel 16

1.3 g BPTI (200 µMol) wurden, wie in Beispiel 5 beschrieben, mit 2.65 g tert.Butylcarbazat (20 mMol) /L.A.Carpino, J.Amer. Chem.Soc. 79, 4427 (1957)/ als nucleophiler Aminoverbindung und 2.5 g N-Äthyl-N'-(3-dimethylaminopropyl)-carbodiimid-hydrochlorid (12 mMol) umgesetzt, und das Reaktionsgemisch wurde entsprechend aufgearbeitet. Man erhielt 1110 mg (85 %) farblose Substanz. Relative elektrophoretische Beweglichkeit: - 1.5 (Cellogel) bzw. - 1.05 (Diskelektrophorese).

Beispiel 17

1 g nach Beispiel 16 erhaltenes BPTI-Derivat wurde, wie in Beispiel 8 angegeben, in Trifluoressigsäure gelöst und die Lösung entsprechend aufgearbeitet. Man erhielt in quantitativer Ausbeute eine farblose Substanz.
Relative elektrophoretische Beweglichkeit: - 1.14 (Cellogel).

Beispiel 18

1.3 g BPTI (200 µMol) und 1.085 g (5 mMol) L-Serin-tert.butyläther-tert.butylester /H.C.Beyerman und J.S.Bontekoe, Proc.Chem. Soc. 1961, 249/ wurden mit Hilfe von 575 mg (3 mMol) N-Äthyl-N'-(3-dimethylaminopropyl)-carbodiimid-hydrochlorid analog zu Beispiel 5 kondensiert. Nach dem Abtrennen von niedrigmolekularen Beimengungen durch Filtration über Sephadex G 10 mit 1 m Essigsäure wurde die Protein enthaltende Fraktion gefriergetrocknet.

Das Lyophilisat löste mit in 7.5 ml wasserfreier Trifluoressigsäure und hielt die Lösung 30 Min. unter Stickstoffatmosphäre bei 22°C. Man verdünnte die Lösung mit 7.5 ml Wasser und fraktionierte sie durch Chromatographie an Sephadex(R) G 50 mit 0.1 m Essigsäure als Elutionsmittel. Ausbeute: 985 mg (76 %). Relative elektrophoretische Beweglichkeit: - 1.62 (Cellogel) bzw. - 1.16 (Diskelektrophorese); Aminosäureanalyse: 1,82 Ser/Mol (BPTI : 1).

Beispiel 19

1.3 g BPTI (200 µMol) und 5.4 g L-Glucosamin (2.5 mMol) wurden, wie in Beispiel 5 beschrieben, mit Hilfe von 2.4 g N-Äthyl-N'-(3-dimethylaminopropyl)-carbodiimid-hydrochlorid (12.5 mMol) in 100 ml Wasser bei pH 5.5 zunächst 1 Std. bei 4°C und weitere 20 Std. bei 22°C umgesetzt. Das Reaktionsgemisch wurde, wie dort beschrieben, aufgearbeitet. Neben 108 mg polymeren Anteilen wurden durch Chromatographie an Sephadex(R) G 50 mit 0,1 m Essigsäure als Elutionsmittel 1020 mg (79 %) farblose Substanz isoliert

Relative elektrophoretische Beweglichkeit: - 1.3 (Cellogel) bzw. - 1.08 (Diskelektrophorese).

Beispiel 20

1 g BPTI (154 µMol) und 2.4 g Trans-4-aminocyclohexyl-1-carbonamid-hydrobromid (10 mMol) wurden, wie in Beispiel 5 beschrieben, mit 1.45 g N-Äthyl-N'-(3-dimethylaminopropyl)-carbodiimid-hydrochlorid (7.5 mMol) umgesetzt und das Reaktionsgemisch wie dort aufgearbeitet.Man erhielt 653 mg (65 %) einer farblosen Substanz.
Relative elektrophoretische Beweglichkeit: - 1.33 (Cellogel) bzw. - 1.06 (Diskolektrophorese);
$/\alpha/_{578}^{20}$ = - 84.3° (c = 0.5, in Wasser).

Beispiel 21

1.3 g BPTI (200 µMol) und 4.4 g Trans-4-aminocyclo-hexan-1-carbonitril-hydrobromid (20 mMol) wurden, wie in Beispiel 5 beschrieben, mit Hilfe von 1.9 g N-Äthyl-N'-(3-dimethylamino-propyl)-carbodiimid-hydrochlorid (10 mMol) umgesetzt, und die Reaktionslösung wurde analog zu Beispiel 5 aufgearbeitet. Man erhielt neben 235 mg polymeren Anteilen 1010 mg farblose Substanz (78 %).
Relative elektrophoretische Beweglichkeit: - 1.43 (Cellogel) bzw. - 1.50 und -1.14 (Doppelbande, Diskelektrophorese);
$/\alpha/_{578}^{20}$ = - 93.5° (c = 0.5, in Wasser).

Beispiel 22

Aus 1.0 g BPTI (154 µMol) und 3.2 g Trans-4-aminocyclo-hexan-1-carboxamidin (10 mMol) wurden durch Kondensation mit 1.9 g N-Äthyl-N'-(3-dimethylaminopropyl)-carbodiimid-hydrochlorid nach Beispiel 5 130 mg polymere Anteile und 900 mg farblose Substanz erhalten.
Relative elektrophoretische Beweglichkeit: - 1.24 (Cellogel) bzw. - 1.08 (Diskelektrophorese);
$/\alpha/_{578}^{22}$ = - 105.7° (c = 0.5, in Wasser).

Beispiel 23

1.3 g BPTI (200 µMol) und 4.0 g L-Cystinamid-dihydrochlorid (12.5 mMol) /I.W.Stapleton und J.M.Swan, Austr.J.Chem. 15, 106 (1962)/ wurden, wie in Beispiel 5 angegeben, unter Zugabe von 1.9 g N-Äthyl-N'-(3-dimethylaminopropyl)-carbodiimid-hydrochlorid (10 mMol) 6 Std. bei pH-Wert 4.75 - eingestellt mit HCl - gehalten. Nach dem üblichen Aufarbeiten erhielt man neben 127 mg polymeren Anteilen (10 %) 1180 mg farblose Substanz (91 %).
Relative elektrophoretische Beweglichkeit: - 1.5 (Cellogel) bzw. - 1.11 (Diskelektrophorese);
$/\alpha/_{578}^{20}$ = - 112.4° (c = 0.4, in Wasser).

Beispiel 24

1.3 g BPTI (200 µMol) und 3.0 g ε-tert.Butyloxycarbonyl-L-lysinamid (12.5 mMol) wurden nach Beispiel 19 mit 1.9 g N-Äthyl-N'-(3-dimethylaminopropyl)-carbodiimid-hydrochlorid bei pH-Wert 4.75 umgesetzt, und die Reaktionslösung wurde entsprechend aufgearbeitet Neben 117 mg polymeren Anteilen (9 %) wurden aus der Monomerenfraktion 1105 mg (81 %) farblose Substanz erhalten.
Relative elektrophoretische Beweglichkeit: - 1.46 (Cellogel) bzw. - 1.11 (Diskelektrophorese); Aminosäureanalyse: 7.34 Lys/Mol (BPTI : 4).

Beispiel 25

550 mg des nach Beispiel 25 dargestellten BPTI-Derivates wurden unter $N_2$-Atmosphäre mit 5 ml absol. Trifluoressigsäure versetzt. Die Reaktionslösung wurde nach 2-stündigem Stehen bei Raumtemperatur mit 50 ml absol. Äther versetzt, der ausgefallene Niederschlag abzentrifugiert und in 10 ml 0.1 m Essigsäure gelöst. Die Lösung wurde durch Filtration über Sephadex G 10 mit 0.1 m Essigsäure entsalzt. Man erhielt nach Chromatographie an Sephadex G 50 473 mg farblose Substanz.
Relative elektrophoretische Beweglichkeit: - 1.38 (Cellogel) bzw. - 1.08 (Diskelektrophorese);
Aminosäureanalyse: 6.24 Lys/Mol (BPTI : 4);

$/\alpha/_{578}^{21}$ = - 107.5° (c = 0.5, in Wasser).

Beispiel 26

1.3 g BPTI (200 µMol) und 5.4 g L-Lysinamid-dihydrochlorid (25 µMol) /S.G.Waley und J.Watson, Biochem.J. 57, 529 (1954)/ wurden, wie für Beispiel 5 beschrieben, mittels N-Äthyl-N'-(3-dimethylaminopropyl)-carbodiimid-hydrochlorid umgesetzt, und die Reaktionslösung wurde entsprechend aufgearbeitet. Man erhielt neben 248 mg polymerem Material (19 %) 1250 mg monomere Substanz.

Relative elektrophoretische Beweglichkeit: - 1,9 bzw. - 1.75 (Doppelbande, Cellogel);
Aminosäureanalyse: 6.3 Lys/Mol (BPTI : 4).

Beispiel 27

Aus 1.3 g BPTI (200 µMol) und 5.0 g L-Argininamid-dihydrochlorid (20 mMol) /L.Zervas, T.T.Otani, M.Winitz und J.P.Greenstein, J.Amer.Chem.Soc. 81, 2878 (1959)/ wurden nach Beispiel 19 mit Hilfe von 1.92 g N-Äthyl-N'-(3-dimethylaminopropyl)-carbodiimid-hydrochlorid (10 mMol) neben 135 mg polymeren Anteilen 1140 mg farblose Substanz (87 %) erhalten.

Relative elektrophoretische Beweglichkeit: -1.65 (Cellogel) bzw. - 1.08 (Diskelektrophorese);
Aminosäureanalyse: 8.3 Arg/Mol (BPTI : 6);

$/\alpha/_{578}^{21} = - 107^{o}$ (c = 0.4, in Wasser).

Beispiel 28

Aus 1.0 g BPTI (154 µMol) und 4.0 g L-Ornithinamid-dihydrochlorid (20 mMol) wurden analog zu Beispiel 5 mit 1.9 g (10 mMol) N-Äthyl-N'-(3-dimethylaminopropyl)-carbodiimid-hydrochlorid bei pH-Wert 4.75 760 mg (76 %) farbloses monomeres Kondensationsprodukt erhalten.

Relative elektrophoretische Beweglichkeit: - 1.57 (Cellogel) bzw. - 1.16 (Diskelektrophorese);

Aminosäureanalyse: 2.18 Orn/Mol;

$/\alpha/^{22}_{578}$ = - 101.5° (c = 0.5, in Wasser).

Beispiel 29

1.3 g BPTI (200 µMol) und 2.27 g L-Histidinamid-dihydrochlorid (10 mMol) /E.L.Smith und D.H.Spackman, J.Biol.Chem. 212, 271 (1955)/ wurden nach dem im Beispiel 5 angegebenen Verfahren unter Verwendung von 0.96 g N-Äthyl-N'-(3-dimethylaminopropyl)-carbodiimid-hydrochlorid bei pH-Wert 4.75 und 4°C umgesetzt. Man erhielt nach dem üblichen Aufarbeiten 80 mg polymere Anteile (6 %) und 1030 mg (79 %) monomere Substanz.

Relative elektrophoretische Beweglichkeit: - 1.72 (Cellogel) bzw. - 1.11 (Diskelektrophorese);
Aminosäureanalyse: 3.95 His/Mol;

$/\alpha/^{21}_{578}$ = - 104.3° (c = 0.5, in Wasser).

Beispiel 30

Aus 1.3 g BPTI (200 µMol) und 506 mg L-Threonyl-L-prolinmethylester-hydrochlorid (2.5 mMol) wurden mit Hilfe von 385 mg N-Äthyl-N'-(3-dimethylaminopropyl)-carbodiimid-hydrochlorid ( 2 mMol) bei pH-Wert 4.8 analog Beispiel 19 1035 mg farblose monomere Substanz dargestellt (80 %).

Relative elektrophoretische Beweglichkeit: - 1.26 (Cellogel) bzw. - 1.08 (Diskelektrophorese);
Aminosäureanalyse: 3,84 Thr/Mol, 6,75 Pro/Mol (BPTI : 3 bzw. 4).

Beispiel 31

Man vereinigte unter Rühren die Lösung von 650 mg BPTI (100 µMol) in 50 ml Wasser mit der Lösung von 1060 mg (2 mMol) L-α,β-Asparagyl-di-(L-serin-tert.butyläther-tert.butylester) in 50 ml absol. Dimethylformamid und setzte schließlich 385 mg (2 mMol) N-Äthyl-N'-(3-dimethylaminopropyl)-carbodiimid-hydrochlorid zu der Mischung. Mit Hilfe von 0.1 n Salzsäure wurde der pH-Wert des Reaktionsgemisches konstant bei 4.85 gehalten. Nach 24 Std. wurden die Lösungsmittel i.Vak. abdestilliert und der Rückstand 1 Std. unter Stickstoffatmosphäre mit 10 ml wasserfreier Trifluoressigsäure gerührt. Nach dem Abdestillieren der Trifluoressigsäure wurde der Rückstand in 5 ml 50 %iger Essigsäure gelöst und die Lösung durch Filtrieren über Sephadex(R) G 10 mit 0,1 m Essigsäure als Elutionsmittel entsalzt. Man erhielt 595 mg (92 %) farblose Substanz.
Relative elektrophoretische Beweglichkeit: - 1.1 u. - 1.35 (Celloge bezw. - 1.47 u. - 1.34 (Diskelektrophorese);
Aminosäureanalyse: 5.78 Asp/Mol und 4.35 Ser/Mol (BPTI : 5 bzw. 1).

Beispiel 32

650 mg BPTI (100 µMol) und 1.23 g Tri-L-glutaminsäure-tetra-tert. butylester (2 m Mol) wurden mit Hilfe von 385 mg N-Äthyl-N'-(3-dimethylaminopropyl)-carbodiimid-hydrochlorid (2 mMol) in einer Mischung von 50 ml Dimethylformamid und 65 ml Wasser nach der im Beispiel 5 angegebenen Methode umgesetzt. Man destillierte die Lösungsmittel i.Vak. ab und rührte den Rückstand 1 Std. bei $20^\circ C$ mit 10 ml Trifluoressigsäure unter Stickstoffatmosphäre. Nach dem Einengen wurde der Rückstand mit 50 %iger Essigsäure als Elutionsmittel über Sephadex(R) G 10 chromatographiert. Man erhielt 643 mg (99 %) farblose monomere Substanz.

Relative elektrophoretische Beweglichkeit: - 0.05(Cellogel) bzw. - 0.82(Diskelektrophorese);
Aminosäureanalyse: 6.44Glu/Mol (BPTI : 3).

Beispiel 33

Analog zu Beispiel 32 wurden 1.3 g BPTI (200 µMol) mit 3.08 g α,β-Asparagyl-bis-α,γ-glutaminsäure-di-tert.butylester (5 mMol) in Gegenwart von 670 mg N-Äthyl-N'-(3-dimethylaminopropyl)-carbodiimid-hydrochlorid (3.5 mMol) in einem Gemisch aus 100 ml Dimethylformamid und 50 ml Wasser kondensiert. Nach der Abspaltung der tert. Butylschutzgruppen analog zu Beispiel 32 wurden 1251 mg farblose Substanz erhalten (96 %).

Relative elektrophoretische Beweglichkeit: - 0.27 u. - 0.05 (Cellogel) bzw. - 0.43, - 0.27 und - 0.18 (Diskelektrophorese);
Aminosäureanalyse: 5.80Asp/Mol und 5.05 Glu/Mol (BPTI : 5 bzw.3).

Beispiel 34

1.033 g in 100 ml Wasser gelöstes Desamino-BPTI-Derivat (160 µMol) erhalten nach Beispiel 34 B, wurden nach erschöpfender Dialyse gegen 0.1 n Salzsäure mit 1.085 g (5 mMol) L-Serin-tert.-butyläther-tert.-butyl-ester (H.C. Beyerman und J.S. Bontekoe, Proc. Chem. Soc. 1961, 249) bei pH-Wert 5.0 unter pH-Kontrolle und Verwendung von 770 mg N-Äthyl-N'-(3-dimethylaminopropyl)-carbodiimid-hydrochlorid (4 mMol), wie in Beispiel 19 beschrieben, zunächst 1 Std. bei 0°C und weitere 20 Std. bei 22°C umgesetzt. Man entsalzte die Reaktionslösung dann durch Chromatographie über Sephadex(R) G 10 mit 0.1 m Essigsäure als Elutionsmittel. Nach dem Gefriertrocknen des Protein enthaltenden Eluates wurden 890 mg (84 %) farblose Substanz erhalten.

Relative elektrophoretische Beweglichkeit: - 1.76 (Cellogel) bzw. - 1.08 (Diskelektrophorese).

720 mg dieser Substanz wurden, wie in Beispiel 18 beschrieben, mit wasserfreier Trifluoressigsäure zum Abspalten der tert. Butylgruppen behandelt, und das Reaktionsprodukt wurde analog zu Beispiel 18 aufgearbeitet. Man erhielt 527 mg (75 %) farblose Substanz.

Relative elektrophoretische Beweglichkeit: - 0.82 (Cellogel) bzw. - 0.69, - 0.62 und - 0.52 (Diskelektrophorese); Aminosäureanalyse: 2,38 Ser/Mol (BPTI : 1).

Beispiel 34 A

500 mg BPTI (77 µMol) wurden in 200 ml eiskalter, sauerstofffreier 0,25 m Essigsäure gelöst. Unter Durchleitung von Stick-

stoff und gutem Rühren tropfte man bei 0-4°C im Verlaufe von 30 Min. 50 ml 2 m Natriumnitritlösung in die Lösung des Inhibitors und hielt das Reaktionsgemisch schließlich 24 Std. unter Stickstoffatmosphäre bei +4°C. Dabei stieg der pH-Wert der Lösung von 3,7 auf 4,1.

Das Reaktionsgemisch wurde nun durch Ultrafiltration in einer Ultrafiltrationszelle (Amicon) mit Hilfe einer Diaflow-UM 05 -Membran eingeengt, mit Wasser verdünnt und erneut konzentriert. Dieser Vorgang wurde mehrfach wiederholt, wobei ein Teil des Reaktionsproduktes schließlich ausfiel. Man versetzte das Retentat mit 0,1 m Ammoniumhydroxidlösung bis zur weitgehenden Lösung des Niederschlags, stellte den pH-Wert der Lösung durch Zugabe von 0,1 m Essigsäure auf 6,8 und fügte soviel Wasser hinzu, daß eine 0,05 m Ammoniumacetat-Konzentration erreicht wurde. Man trennte eine kleine Menge ungelöstes Material durch Zentrifugieren ab und chromatographierte die überstehende Lösung an einer mit 0,05 m Ammoniumacetatlösung pH 6,8 äquilibrierten SP-Sephadex® C-25-Säule (2,5 x 80 cm). (Sephadex® = quervernetztes Dextran). Die Säule wurde zunächst mit 500 ml 0,05 m Ammoniumacetatlösung pH 6,8 und anschließend mit 500 ml 0,5 m Ammoniumacetatlösung des gleichen pH-Wertes eluiert. Das Elutionsprofil ist in Figur 1 wiedergegeben. Die einzelnen Fraktionen A bis F (vgl. Figur 1) wurden gefriergetrocknet und die Lyophilisate teils durch Chromatographie an Biogel P 2 mit 0,1 m Ammoniumhydroxid als Elutionsmittel, teils durch Ultrafiltieren entsalzt. Die Ausbeuten und spektroskopischen Daten der aus den einzelnen Fraktionen erhaltenen Präparate sind in Tab. A zusammengestellt und die Resultate der

quantitativen Aminosäureanalysen in Tab. C wiedergegeben. In Figur ist auf der Ordinate die Estinktion in Prozent und auf der Abszisse die Bezeichnung der jeweiligen Fraktion A bis F aufgeführt. Beim Punkt W wird das Elutionsmittel gewechselt (0,5 Ammoniumacetatlösung anstelle von 0,5 m Ammoniumacetatlösung).

Beispiel 34 B

500 mg BPTI wurden, wie in Beispiel 34 A beschrieben, mit Natriumnitrit in essigsaurer Lösung umgesetzt. Nach 24 Stunden wurde der pH-Wert durch Zugabe von konz. wäßrigem Ammoniak auf 9,0 gestellt, die Lösung, wie in Beispiel 34 A beschrieben, durch Ultrafiltration entsalzt. Höhermolekulare Anteile wurden durch Chromatographie an einer Sephadex® G-50-Säule (2,5 x 100 cm) mit 0,1 m Ammoniak-Lösung abgetrennt. Nach dem Gefriertrocknen wurde der Rückstand in 10 ml 0,1 m wäßrigem Ammoniak gelöst und mit 0,1 m Essigsäure auf pH 6,8 gestellt. Die klare Lösung wurde nun, wie in Beispiel 34 A angegeben, auf eine SP-Sephadex® C-25-Säule (2,5 x 70 cm) aufgetragen und zunächst mit 500 ml 0,05 m Ammoniumacetatlösung pH 6,8 eluiert, gefolgt von 500 ml 0,5 m Ammoniumacetatlösung pH 6,8 und schließlich 200 ml 0,1 m wäßrigem Ammoniak. Das Elutionsprofil ist mit dem nach Beispiel 34 A erhaltenen vergleichbar. Die Ausbeuten und spektroskopischen Daten sind für die einzelnen Fraktionen in Tab. B wiedergegeben.

Tabelle A: Fraktionierung des nach Beispiel 34 A dargestellten Desamino-BPTI-Gemisches an SP-Sephadex® C-25

| Fraktions-Nr. | Bezeichnung | Menge (mg) | $E\frac{280\ nm}{425\ nm}$ |
|---|---|---|---|
| 21 - 30 | A | 224 | 5,53 |
| 31 - 40 | B | 73 | 8,57 |
| 41 - 52 | C | 37 | 6,15 |
| 53 - 63 | D | 87 | 4,61 |
| 75 -105 | E | 67 | 16,60 |
| 106 -140 | F | 12 | 13,25 |

Tabelle B: Fraktionierung des nach Beispiel 34 B dargestellten Desamino-BPTI-Gemisches an SP-Sephadex® C-25

| Fraktions-Nr. | Bezeichnung | Menge (mg) | $E\frac{280\ nm}{425\ nm}$ |
|---|---|---|---|
| 19 - 27 | A | 7 | 9,0 |
| 28 - 42 | B | 22 | 13,3 |
| 43 - 48 | C | 177 | 17,4 |
| 49 - 51 | D | 44 | 21,2 |
| 52 - 59 | E | 9 | 15,5 |
| 113 - 127 | F | 185 | 22,1 |

Tabelle C: Bei der Aminosäureanalyse von Desamino-BPTI-Derivaten gemäß Bsp. 34 A und 34 B ermittelte Gehalte an charakteristischen Aminosäuren

| Bezeichnung der Substanz | Herstellung, beschrieben in Beispiel | Mol Aminosäure-Rest / Mol BPTI-Derivate [1)] Glycin [2)] | Alanin [2)] | Tyrosin | Lysin | Arginin |
|---|---|---|---|---|---|---|
| BPTI [3)] | | 6,09 | 6,00 | 3,80 | 4,08 | 6,01 |
| Desamino-BPTI-Derivat, Fr. A | 34 A | 6,18 | 6,66 | 2,11 | 0,27 | 3,51 |
| " " " B | | 6,08 | 6,35 | 2,83 | 0 | 4,27 |
| " " " C | | 6,19 | 6,80 | 2,61 | 0 | 3,95 |
| " " " D | | 6,81 | 5,81 | 2,53 | 0,18 | 4,44 |
| " " " E | | 6,58 | 6,24 | 3,32 | 0 | 4,40 |
| " " " F | | 6,31 | 5,92 | 2,98 | 0,25 | 4,50 |
| Desamino-BPTI-Derivat, Fr. A | 34 B | 6,32 | 6,08 | 3,51 | 0,30 | 3,61 |
| " " " B | | 5,98 | 5,61 | 3,76 [4)] | 0,26 | 3,84 |
| " " " C | | 6,01 | 5,62 | 4,55 [4)] | 0,21 | 4,81 |
| " " " D | | 6,49 | 5,93 | 3,74 | 0,14 | 4,64 |
| " " " E | | 6,82 | 5,58 | 4,72 [4)] | 1,06 | 4,88 |
| " " " F | | 6,53 | 5,58 | 3,71 | 0,92 | 3,92 |

0001774

1) Das Molekulargewicht der Derivate wurde zu 6511 wie für nativen BPTI angenommen.

2) Der Alanin- bzw. Glycingehalt dienen als interner Standard. Je nach den Elutionsbedingungen bei der Aminosäureanalyse werden unbekannte mit Ninhydrin anfärbbare Substanzen zusammen mit Glycin und/oder Alanin eluiert, die dann höhere Glycin- und/oder Alanin-Gehalte vortäuschen.

3) Zum Vergleich wurde eine Aminosäureanalyse von BPTI mit aufgenommen. Die theoretischen Werte der betreffenden Aminosäuren betragen: Gly und Ala = 6,0; Tyr = 4.0; Lys = 4,0; Arg. = 6,0.

4) Ein Desaminierungsprodukt von Lysin wird unmittelbar vor Tyrosin eluiert. Deshalb wird der Tyrosin-Peak im Chromatogramm entweder unsymmetrisch oder begleitet von einem nicht ganz aufgelösten Gipfel gefunden.

Beispiel 35

Die Lösung von 550 mg (85 µMol) guanidiertem BPTI /B.Kassell und R.B.Chow, Biochemistry 5, 3449-3453 (1960); J.Chauvet und R.Acher, Biochem.Biophys.Res.Comm. 27, 230-235 (1967)/ und 410 mg (1.9 mMol) L-Serin-tert.butyläther-tert.butylester /H.C.Beyerman und J.S.Bontekoe, Proc.Chem.Soc. 1961, 249/ in 55 ml Wasser wurde unter pH-Kontrolle bei pH-Wert 4.9 mit 163 mg N-Äthyl-N'-(3-dimethylaminopropyl)-carbodiimid-hydrochlorid (850 µMol), wie in Beispiel 19 beschrieben, umgesetzt. Man entsalzte die Reaktionslösung an Sephadex G 10 - 5 x 100 cm Säule durch Filtrieren mit 50 %iger Essigsäure als Elutionsmittel. Nach dem Gefriertrocknen des Protein enthaltenden Eluates wurde die Lösung der Substanz in 7.5 ml wasserfreier Trifluoressigsäure 30 Min. unter Stickstoffatmosphäre bei Raumtemperatur gehalten. Die mit 5 ml Wasser verdünnte Lösung wurde durch Filtration über Sephadex G 10 entsalzt. Durch Chromatographie an Sephadex G 50 mit 0.1 m Essigsäure wurden 51 mg polymere Anteile abgetrennt und 248 mg farblose Substanz erhalten (45 %).

Relative elektrophoretische Beweglichkeit: - 1.16 (Cellogel) bzw. - 1.08 (Diskelektrophorese).

Aminosäureanalyse: 2,26 Ser/Mol, 3,60 Homoarg/Mol
BPTI: 1 Ser; 4 Lys

Beispiel 36

Analog zu Beispiel 38 wurden 200 mg guanidierter BPTI (31 µMol) mit 250 mg N-Äthyl-N'-(3-dimethylaminopropyl)-carbodiimid-hydrochlorid (1.3 mMol) und 670 mg Di-L-glutaminsäure-tri-tert.butylester (1.5 mMol) umgesetzt. Die Reaktionslösung wurde auf 10 ml i.Vak. eingeengt, und der pH-Wert der Lösung wurde mit konz.

Salzsäure auf 1.0 eingestellt. Man hielt das Gemisch anschließend 10 Std. bei Raumtemperatur und filtrierte die Lösung zum Abtrennen der Salze und der niedrigmolekularen Beimengungen über Sephadex G 10 mit 0.1 m Essigsäure als Elutionsmittel. Man erhielt 175 mg farblose Substanz (87 %).

Relative elektrophoretische Beweglichkeit: - 0.60 (Cellogel) bzw. - 0.62 und 0.50 (Diskelektrophorese);
Aminosäureanalyse: 3,95 Glu/Mol (BPTI : 3).

Beispiel 37

Die Lösung von 1.3 g (200 µMol) Desamido-BPTI - erhalten durch 25 tägiges Stehenlassen von nativem BPTI in 2 n Salzsäure bei 22°C und anschließende Dialyse und Gefriertrocknung - und 1.2 g L-Serin-tert.butyläther-tert.butylester (5.5 mMol) /H.C.Beyerman und J.S.Bontekoe, Proc.Chem.Soc. 1961, 249/ in 100 ml Wasser wurde analog zu Beispiel 8 mit 960 mg N-Äthyl-N'-(3-dimethylaminopropyl)-carbodiimid-hydrochlorid (5 mMol) umgesetzt. Die nach 24 Std. analog zu Beispiel 8 durchgeführte Aufarbeitung ergab 1100 mg (84 %) farblose Substanz.

Relative elektrophoretische Beweglichkeit: - 1.16 (Cellogel).

950 mg dieser Substanz wurden zur Abspaltung der tert.Butylgruppen in Analogie zu Beispiel 18 mit wasserfreier Trifluoressigsäure behandelt und die Lösung, wie dort beschrieben, aufgearbeitet. Nach Chromatographie an Sephadex G 50 mit 0.1 m Essigsäure als Elutionsmittel wurden neben 53 mg polymeren Anteilen 760 mg (75 %) farblose Substanz erhalten.

Relative elektrophoretische Beweglichkeit: - 0.98 (Cellogel) bzw. - 0.90 (Diskelektrophorese);
Aminosäureanalyse: 3,61 Ser/Mol (BPTI : 1).

Beispiel 38

490 mg (75 µMol) der in Beispiel 37 als Ausgangsmaterial verwendeten Desamido-BPTI-Präparation wurde entsprechend der in Beispiel 37 beschriebenen Umsetzung mit L-Glutaminsäure-di-tert. butylester umgesetzt. Der Ansatz wurde wie dort beschrieben aufgearbeitet. Man isolierte 485 mg farblose Substanz (99 %).

Relative elektrophoretische Beweglichkeit: - 1.52 (Cellogel).

480 mg dieser Substanz wurden unter Stickstoffatmosphäre durch Behandeln mit Trifluoressigsäure, analog zu Beispiel 21, debutyliert. Das Reaktionsgemisch wurde entsprechend aufgearbeitet. Man erhielt so 470 mg (96 %) farblose Substanz.

Relative elektrophoretische Beweglichkeit: - 0.92 (Cellogel) bzw. - 0.88 (Diskelektrophorese);
Aminosäureanalyse: 6.88 Glu/Mol (BPTI : 3).

Beispiel 39

Aus 650 mg Desamido-BPTI (100 µMol) (vgl. Beispiel 37 und 1420 mg Tri-L-glutaminsäure-tetra-tert.butylester (2.25 mMol) wurden mit Hilfe von 405 mg N-Äthyl-N'-(3-dimethylaminopropyl)-carbodiimid-hydrochlorid (2.1 mMol) nach dem im Beispiel 38 beschriebenen Verfahren 418 mg Kondensationsprodukt gewonnen. (64%).

Relative elektrophoretische Beweglichkeit: - 0,88 (Cellogel) bzw. -0,82/0,17 (Diskelektrophorese);
Aminosäureanalyse: 9.54 Glu/Mol (BPTI : 3).

Beispiel 40

520 mg Mono-nitro-BPTI (80 µMol) /B.Meloun, I.Frič und F.Šorm, Europ.J.Biochem. 4, 112 (1968)/ und 650 mg Glutaminsäure-di-tert.butylester (2.5 mMol) wurden wie in Beispiel 10 beschrieben mit Hilfe von N-Äthyl-N'-(3-dimethylaminopropyl)-carbodiimid-hydrochlorid umgesetzt. Die tert. Butylgruppen wurden analog zu Beispiel 31 durch Behandeln mit Salzsäure bei pH-Wert 0.5 - 1.0 abgespalten. Die Reaktionslösung wurde durch Filtration über Sephadex G 10 mit 0.1 m Essigsäure als Elutionsmittel entsalzt. Man erhielt 480 mg gelbe Substanz (92 %).

Relative elektrophoretische Beweglichkeit: - 0.89 (Cellogel) bezw. - 0.82 und - 0.67 (Diskelektrophorese);
Aminosäureanalyse: 4.01 Glu/Mol (BPTI : 3);

$$\frac{E_{278}}{E_{430}} = 2.03 \text{ (0.1 m Tris-Puffer, pH 7.8).}$$

Beispiel 41

Aus 490 mg Dinitro-BPTI (75 µMol) /B.Meloun, I.Frič und F.Šorm, Europ.J.Biochem. 4, 112 (1968)/ und 1100 mg L-Serin-tert.butyläther-tert.butylester (5 mMol) wurden durch Kondensation mit Hilfe von 670 mg (3.5 mMol) N-Äthyl-N'-(3-dimethylaminopropyl)-carbodiimid-hydrochlorid und anschließende Abspaltung der tert. Butylgruppen, nach dem im Beispiel 40 angegebenen Verfahren 418 mg gelbe Substanz (85 %) erhalten.

Relative elektrophoretische Beweglichkeit: - 0.94 (Cellogel) bzw. - 0.78 und - 0.59 (Diskelektrophorese);
Aminosäureanalyse: 2.40 Ser/Mol (BPTI : 1);

$$\frac{E_{278}}{E_{430}} = 1.44 \text{ (0.1 m Tris-Puffer, pH 7.8).}$$

Beispiel 42

Analog zu Beispiel 40 wurden bei der Verwendung von 1300 mg Dinitro-BPTI (200 µMol) und 780 mg L-Glutaminsäure-di-tert. butylester (3 mMol) 881 mg gelbe Substanz (68 %) erhalten. Relative elektrophoretische Beweglichkeit: - 1.31 (Cellogel) bzw. - 1.16 (Diskelektrophorese);

860 mg der Substanz wurden analog zu Beispiel 11 1 Std. mit 10 ml wasserfreier Trifluoressigsäure behandelt und das Reaktionsprodukt wie dort isoliert. Nach Chromatographie an Sephadex G 50 mit 0.1 m Essigsäure als Elutionsmittel erhielt man 550 mg gelbe Substanz (64 %).

Relative elektrophoretische Beweglichkeit: - 1.12 (Cellogel) bzw. - 0.95 (Diskelektrophorese);
Aminosäureanalyse: 4.03 Glu/Mol (BPTI : 3);

$$\frac{E_{278}}{E_{430}} = 1.47 \text{ (0.1 m Tris-Puffer, pH 7.8).}$$

Beispiel 43

650 mg (100 µMol) durch Dialyse gegen 0.1 n Salzsäure bei 4°C von Ammonium- und Acetat-Ionen befreiter guanidierter BPTI /B.Kassell und R.B.Chow, Biochemistry 5, 3449 (1966)/, 135 mg N-Hydroxybenztriazol (1 mMol) und 260 mg L-Glutaminsäure-di-tert. butylester (4 mMol) wurden mit 10 ml absol. Dimethylformamid versetzt. Bei 4°C setzte man 206 mg Dicyclohexylcarbodiimid zu der Suspension und rührte das Reaktionsgemisch zunächst 1 Std. bei 4°C und weitere 24 Std. bei Raumtemperatur. Dann wurde das Lösungsmittel i.Vak. bei 50°C abdestilliert und der Rückstand mit 10 ml wasserfreier Trifluoressigsäure 30 Min. unter Stickstoffatmosphäre gerührt. Das Reaktionsgemisch wurde i.Vak. eingeengt und der Rückstand mit 10 ml 50 %iger Essigsäure versetzt. Man zentrifugiert das ungelöste Material ab und filtrierte die überstehende Lösung über Sephadex G 10 mit 50 %iger Essigsäure als Elutionsmittel.

Nach der im Beispiel 1 beschriebenen Aufarbeitung erhielt man 540 mg (83 %) farblose Substanz.

Relative elektrophoretische Beweglichkeit: - 0.56 u. 0 (Celloge: bzw. - 0.84 und - 0.74 (Diskelektrophorese); Aminosäureanalyse: 7.27 Glu/Mol; 1.21 Lys/Mol (BPTI: 3 Glu bzw. 4 Lys)

Beispiel 44

650 mg (100 µMol) aus Dinitro-BPTI /B. Meloun, I. Frič und F. Šorm, Europ. J. Biochem. 4, 112 (1968)/ analog zu Beispiel 44 A erhaltener Bis-3-aminotyrosyl-BPTI wurden, wie für Beispiel 5 beschrieben, in Gegenwart von N-Äthyl-N'-(3-dimethylaminopropyl)-carbodiimid-hydrochlorid mit L-Glutam säure-di-tert.butylester kondensiert und entsprechend aufgearbei Man erhielt 509 mg (78 %) farbloses Kondensationsprodukt.

Relative elektrophoretische Beweglichkeit: - 1.0 (Cellogel) bzw. - 0.95 (Diskelektrophorese); Aminosäureanalyse: 4.45 Glu/Mol; 1.55 Tyr/Mol (BPTI: 3 Glu bzw. 4 T

Beispiel 44 A

650 mg (100 µ Mol) eines Gemisches von nitriertem BPTI /B.Meloun, I. Fric und F. Sorm, Europ. J. Biochem. 4, 112 (1968)/ wurden in 25 ml m Tris-(hydroxymethyl)-amino-methan-Puffer, pH 7,5 gelöst. Unter gutem Rühren fügte man in zwei Anteilen je 300 mg Natriumdithionit im Abstand von 10 Minuten zu der Lösung und versetzte das Gemisch 10 Minuten nach der letzten Zugabe mit Essigsäure, bis ein pH-Wert von 5,0 erreicht wurde. Zur Entsalzung wurde das Reaktionsgemisch über Sephadex G 10 mit 0,1 m Essigsäure als Elutionsmittel filtriert. Die Protein enthaltenden Eluate wurden gefriergetrocknet. Man

löste das Lyophilisat in 5 ml n Salzsäure und versetzte die Lösung nach dem Abkühlen auf 4°C mit einer Lösung von 14 mg Natriumnitrit (203 µMol) in 100 µl Wasser unter gutem Rühren. Nach 10 Minuten fügte man 10 mg feste Amidosulfonsäure zu der Lösung und nach weiteren 5 Minuten eine vorgekühlte Lösung von 94 mg Phenol (1 mMol) in 5 ml n Natronlauge (pH 8,2). Man rührte die gelbe Suspension unter Zugabe von Natronlauge, bis ein pH-Wert von 9,0 erreicht wurde. Im Verlaufe von 30 Minuten wurde eine rote Lösung erhalten. Nach Zugabe von Essigsäure bis pH 4,0 wurde die Reaktionslösung durch Chromatographie an Sephadex G 10 entsalzt. Anschließend wurden die höhermolekularen Anteile durch Chromatographie der Protein enthaltenden Eluate an Sephadex G 50 mit 0,1 m Essigsäure als Elutionsmittel abgetrennt. Man erhielt schließlich 425 mg gelborangefarbene Substanz.

Beispiel 45

1.3 g BPTI (200 µMol) wurden gemäß Beispiel 50 mit 2 x 100 µl Citraconsäureanhydrid (2 mMol) umgesetzt. Zu der eisgekühlten Lösung des Citraconylderivates fügte man 4.35 g (20 mMol) L-Serin-tert.butyläther-tert.butylester und nach Einstellen eines pH-Wertes von 5.2 3.85 g N-Äthyl-N'-(3-dimethylaminopropyl)-carbodiimid-hydrochlorid. Man hielt die Reaktionsmischung 3 Std. bei 22°C, wobei eine milchige Suspension entstand. Man stellte den pH-Wert dieser Suspension mit konz.Salzsäure auf 1.0 ein und rührte sie 20 Std. Dabei entstand eine klare Lösung die analog zu Beispiel 1 aufgearbeitet wurde. Man erhielt 1050 mg (81 %) farbloses Lyophilisat.

Relative elektrophoretische Beweglichkeit: - 0.56 (Cellogel) bzw. - 0.98 und - 0.86 (Diskelektrophorese);

Aminosäureanalyse: 2.96 Ser/Mol (BPTI : 1).

BAD ORIGINAL

Desaminierung von Carboxyl-modifizierten BPTI-Derivaten

Beispiel 46

320mg des nach Beispiel 2 dargestellten BPTI-Derivats wurden in 100 ml Sauerstoff-freier 0,25 m Essigsäure gelöst. In diese Lösung wurden unter Rühren und Aufrechterhalten einer Stickstoffatmosphäre bei 4°C innerhalb von 30 Minuten 25 ml einer 2 m Natriumnitritlösung zufließen gelassen. Das Reaktionsgemisch wurde weitere 24 Stunden bei 4°C gerührt. Dann stellte man den pH-Wert der Lösung mit konzentriertem wäßrigen Ammoniak auf 8,0 ein und engte das Volumen der Lösung mit Hilfe einer Ultrafiltrationszelle auf 10 ml ein. Man entsalzte die Lösung durch Filtration über Sephadex® G 10 mit 0,1 m Ammoniak als Elutionsmittel. Beim Lyophilisieren des Protein enthaltenden Eluats wurden 230 mg (72 %) gelbliche Substanz erhalten.

Relative elektrophoretische Beweglichkeit: - 0,07 (Cellogel);
Aminosäureanalyse: vgl. Tab. 1

Beispiel 47

Wie im Beispiel 46 beschrieben, wurden 600 mg des nach Beispiel 30 dargestellten BPTI-Derivates unter Stickstoffatmosphäre mit Natriumnitritlösung in essigsaurer Lösung desaminiert. Nach dem üblichen Aufarbeiten wurden 368 mg farblose Substanz erhalten.

Relative elektrophoretische Beweglichkeit: - 0.26 (Cellogel) bzw. - 0.67, - 0.54 und - 0.43 (Diskelektrophorese).

Beispiel 48

325 mg nach Beispiel 2 gewonnenes BPTI-Derivat wurden mit aus 10 mg 5-Aminotetrazol (115 µMol) dargestelltem Diazoniumsalz bei pH-Wert 8,5 umgesetzt. Das Desaminierungsprodukt wurde durch entsprechende Aufarbeitung des Reaktionsgemisches isoliert. Nach dem Filtrieren des Reaktionsproduktes über Sephadex G 10 mit 0.05 m Ammoniumbicarbonatlösung als Elutionsmittel wurden 205 mg (63 %) farblose Substanz erhalten.

Relative elektrophoretische Beweglichkeit: - 1.28 und - 1.5 (Doppelbande; Cellogel) bzw. - 1.13 und - 1.43 (Diskelektrophorese); Aminosäureanalyse: vgl. Tab.1.

Kupplung von Carboxyl-modifizierten BPTI-Derivaten mit Diazoniumverbindungen

Beispiel 49

Die Lösung von 50 mg 3,5-Dichloranilin (310 µMol) in 1 ml einer 1 + 1 Mischung von Essigsäure udd 80 %iger Phosphorsäure wurde bei 4°C unter Rühren mit einer Lösung von 50 mg Natriumnitrit (360 µMol) in 100 µl Wasser versetzt. Nach 10 Min. fügte man 30 mg Harnstoff hinzu und vereinigte die Reaktionslösung nach weiteren 10 Min. mit einer eiskalten Lösung von 500 mg des nach Beispiel 11 dargestellten BPTI-Derivates in 7.5 ml 0.1 m Natriumtetraboratlösung, pH 9,2. Man stellte den pH-Wert der Reaktionsmischung durch Zugabe von eiskalter 8 n Natronlauge auf 8.0 ein, wobei ein Niederschlag entstand. Nach 1-stündigem Rühren bei 4°C wurden 50 mg Phenol zu der Mischung gegeben und diese

BAD ORIGINAL

schließlich mit 15 ml Eisessig verdünnt. Die so erhaltene klare, intensiv rote Lösung wurde zum Abtrennen der niedermolekularen Anteile mit 50 %iger Essigsäure als Elutionsmittel über Sephadex G 10 filtriert. Aus dem Protein enthaltenden Eluat wurden 300 mg (60 %) orangefarbene Substanz isoliert.

Relative elektrophoretische Beweglichkeit: 0.0 (Cellogel) bzw. - 1.0, - 0.92, - 0.67 (Diskelektrophorese);
$E_{328}^{1\,cm}$ = 15 (bei 6.5 g/l, 0.1 m Tris-Puffer, pH 7.2).

Beispiel 50

Zur Lösung von 325 mg nach Beispiel 2 erhaltenem BPTI-Derivat (50 µMol) wurden unter Rühren bei 4°C 100 µl Citraconsäureanhydrid (1.1 mMol) zufliessen gelassen, wobei der pH-Wert der Lösung durch Zugabe von 8 n Natronlauge bei 8.0 gehalten wurde. Das Reaktionsgemisch wurde anschließend weitere 30 Min. bei 4°C gerührt und mit einer nach Beispiel 49 aus 32 mg 3,5-Dichloranilin hergestellten Lösung der entsprechenden Diazoniumverbindung vereinigt. Die Kupplung wurde analog zu Beispiel 49 bei pH 8.0 durchgeführt.

Zur Abspaltung der Citraconylgruppen versetzte man das Reaktionsgemisch mit 20 ml Eisessig und fügte zusätzlich konz. Salzsäure hinzu bis das Reaktionsgemisch einen pH-Wert von 3.5 erreicht hatte. Nach 24-stündigem Stehen bei 20°C wurde die Lösung zum Abtrennen der niedermolekularen Komponenten mit 50 %iger Essigsäure über Sephadex G 10 filtriert. 10 % polymere Reaktionsprodukte wurden durch Chromatographie an Sephadex G 50 abgetrennt. Beim Gefriertrocknen der Monomeren-Fraktion wurden 235 mg orangefarbene Substanz (72.5 %) erhalten.

Relative elektrophoretische Beweglichkeit: - 0.05 und - 0.90 (Cellogel) bzw. - 1.0 (Diskelektrophorese);
$E_{328}^{1\,cm}$ = 24.5 (bei 6.5 g/l, 0.1 m Essigsäure); Aminosäureanalyse: vgl. Tab. 1.

Beispiel 51

Aus 500 mg nach Beispiel 8 hergestelltem BPTI-Derivat (77 µMol) wurden, wie in Beispiel 49 beschrieben, durch Kuppeln mit der aus 50 mg 3,5-Dichloranilin hergestellten Diazoniumverbindung nach dem Abtrennen der niedermolekularen Begleitstoffe durch Filtration der Reaktionslösung über Sephadex G 10 mit 50 %iger Essigsäure 361 mg (72 %) orangefarbene Substanz erhalten.
Relative elektrophoretische Beweglichkeit: - 0.0 (Cellogel) bzw. - 0.21, - 0.42, - 0.52, - 0.75 und - 0.98 (Diskelektrophorese);
$E_{325}^{1\,cm}$ = 8.0 (bei 6.5 g/l, 50 %ige Essigsäure).

Beispiel 52

500 mg nach Beispiel 13 erhaltenes BPTI-Derivat (77 µMol) wurden analog zu Beispiel 50 citraconyliert und, wie dort beschrieben, zur Kupplung mit einer Lösung von aus 3,5-Dichloranilin hergestellter Diazoniumverbindung umgesetzt. Nach der Abspaltung der Citraconylgruppen wurden analog zu Beispiel 50 325 mg (65 %) braune Substanz erhalten.

Relative elektrophoretische Beweglichkeit: 0.0 (Cellogel) bzw. - 1.05 (Diskelektrophorese);
$E_{328}^{1\,cm}$ = 23.5 (bei 6.5 g/l, 50 %ige Essigsäure).

Beispiel 53

Aus 500 mg nach Beispiel 10 gewonnenem BPTI-Derivat (77 µMol) erhielt man analog zu Beispiel 49 385 mg (77 %) einer gelborangen Substanz.

Relative elektrophoretische Beweglichkeit: 0.0 (Cellogel) bzw. - 1.0 und - 0.85 (Diskelektrophorese);
$E_{328}^{1\,cm}$ = 14.0 (bei 6.5 g/l, 50 %ige Essigsäure).



BAD ORIGINAL

Beispiel 54

Aus 250 mg nach Beispiel 15 hergestelltem BPTI-Derivat (38 µMol) wurden analog zu Beispiel 49 105 mg (42 %) orangegelbe Substanz gewonnen.

Relative elektrophoretische Beweglichkeit: 0.0 (Cellogel) bzw. - 1.04 (Diskelektrophorese);

$E^{1\,cm}_{328}$ = 13.5 (bei 6.5 g/l, 50 %ige Essigsäure);

Aminosäureanalyse: vgl. Tab. 1.

Beispiel 55

Aus 500 mg nach Beispiel 4 hergestelltem BPTI-Derivat wurden analog zu Beispiel 49 234 mg gelborange-farbenes Azoderivat gewonnen (47 %).

Relative elektrophoretische Beweglichkeit: 0.0 (Cellogel) bzw. - 1.0, - 0.82 und - 0.67 (Diskelektrophorese);

$E^{1\,cm}_{328}$ = 10.5 (bei 6.5 g/l, 0.1 m Tris-Puffer, pH 7.5).

Beispiel 56

Aus 250 mg des nach Beispiel 29 erhaltenen BPTI-Derivates wurden nach dem im Beispiel 50 beschriebenen Verfahren durch Kuppeln mit 3,5-Dichlorbenzoldiazoniumverbindung 208 mg (83 %) ziegelrotes Azoderivat hergestellt.

Relative elektrophoretische Beweglichkeit: - 0.05 (Cellogel) bzw. - 1.11 (Diskelektrophorese);

$E^{1\,cm}_{328}$ = 36.0 (bei 6.5 g/l, 50 %ige Essigsäure);

Aminosäureanalyse: vgl. Tab. 1.

Beispiel 57

Aus 500 mg nach Beispiel 6 erhaltenem BPTI-Derivat wurden in Analogie zu Beispiel 49 363 mg (72 %) orangefarbenes Azoderivat hergestellt und isoliert.

Relative elektrophoretische Beweglichkeit: 0.0 (Cellogel) bzw. - 0.85 (Diskelektrophorese);

$E^{1\,cm}_{328}$ = 11.8 (bei 6.5 g/l, 50 %ige Essigsäure);

Aminosäureanalyse: vgl. Tab. 1.

Beispiel 58

Aus 500 mg nach Beispiel 27 dargestelltem BPTI-Derivat wurden analog zu Beispiel 49 84 mg (17 %) polymere und 270 mg (54 %) monomere orangefarbene Azoverbindungen erhalten.

Relative elektrophoretische Beweglichkeit: - 0.05 (Cellogel) bzw. - 1.11 (Diskelektrophorese);

$E^{1\,cm}_{328}$ = 9.05 (bei 6.5 g/l, 50 %ige Essigsäure);

Aminosäureanalyse: vgl. Tab. 1.

Beispiel 59

Aus 250 mg nach Beispiel 28 hergestelltem BPTI-Derivat wurden, wie in Beispiel 50 beschrieben, durch Citraconylierung und Umsetzung des Reaktionsproduktes mit diazotiertem 3,5-Dichloranilin nach Abspalten der Citraconyl-Reste 192 mg braunes Azo-BPTI-Derivat gewonnen (77 %).

Relative elektrophoretische Beweglichkeit: 0.0 (Cellogel) bzw. - 1.05 (Diskelektrophorese);

$E^{1\,cm}_{328}$ = 14.75 (bei 6.5 g/l, 0.1 m Tris-Puffer, pH 7.5).

Beispiel 60

Aus 125 mg nach Beispiel 24 erhaltenem BPTI-Derivat wurden in Analogie zu Beispiel 49 147 mg orangefarbene Substanz erhalten.

Proteingehalt: 75 %.

Relative elektrophoretische Beweglichkeit: - 0.0 (Cellogel) bzw. - 1.02 (Diskelektrophorese);

$E_{328}^{1\,cm}$ = 14.45 (bei 6.5 g/l, 0.1 m Essigsäure).

Beispiel 61

Aus 250 mg nach Beispiel 21 gewonnenem BPTI-Derivat erhielt man analog zu dem in Beispiel 50 beschriebenen Verfahren nach Citraconylierung durch Kuppeln des Reaktionsproduktes mit aus 3.5-Dichloranilin hergestellter Diazoniumverbindung und Abspalten der Citraconyl-Reste 202 mg orangefarbenes Azoderivat.

Relative elektrophoretische Beweglichkeit: - 0.13 (Cellogel) bzw. - 0.96 (Diskelektrophorese);

$E_{328}^{1\,cm}$ = 15.8 (bei 6.5 g/l, 0.1 m Essigsäure).

Beispiel 62

250 mg (38 µMol) des nach Beispiel 25 erhaltenen BPTI-Derivates wurden, wie für Beispiel 50 beschrieben, citraconyliert, und das Citraconylderivat wurde bei pH 8.5 analog zu der in Beispiel 50 beschriebenen Methode mit einer Lösung von aus 28 mg (330 µMol) 3-Amino-1,2,4-triazol in 5 ml n-Salzsäure mit 25 mg Natriumnitrit (365 µMol) wie üblich dargestellter Diazoniumverbindung gekuppelt. Nach dem Abspalten der Citraconylgruppen, analog Beispiel 50,

wurde das Reaktionsprodukt an Sephadex G 10 mit 50 %iger Essigsäure als Laufmittel entsalzt. Man isolierte 178 mg (71 %) eines rötlich-braunen Azoderivates.

Relative elektrophoretische Beweglichkeit: - 0.74 und - 1.08 (Cellogel) bzw. - 1.06 (Hauptbande, Diskelektrophorese);

$E_{328}^{1\,cm}$ = 22.6 (bei 6.5 g/l, 0.1 m Essigsäure).

Beispiel 63

Analog zu Beispiel 62 wurden 250 mg BPTI-Derivat aus Beispiel 25 (35 µMol), wie in Beispiel 50 beschrieben, citraconyliert und das Reaktionsprodukt bei einem pH-Wert von 8.5 mit aus 27 mg 5-Aminotetrazol (320 µMol) und 25 mg Natriumnitrit in 5 ml 1 n Salzsäure dargestelltem Diazoniumsalz gekuppelt. Die Reaktionslösung wurde aufgearbeitet, wie für Beispiel 54 beschrieben. Man erhielt 183 mg orangefarbenes Azoderivat.

Relative elektrophoretische Beweglichkeit: 0.0 (Cellogel) bzw. - 1.04, - 0.94 sowie - 0.84 (Diskelektrophorese);

$E_{328}^{1\,cm}$ = 5.2 (bei 6.5 g/l, 0.1 m Essigsäure).

Beispiel 64

250 mg des nach Beispiel 20 erhaltenen BPTI-Derivates wurden in 5 ml 0.1 m Natriumtetraboratlösung gelöst und nach der im Beispiel 50 angegebenen Methode citraconyliert. Die so erhaltene Verbindung wurde, wie in Beispiel 50 beschrieben, zur Kupplung bei pH 8.0 mit der Lösung von aus 25 mg 3,5-Dichloranilin erhaltene Diazoniumverbindung umgesetzt. Nach dem Entsalzen des Reaktionsproduktes und anschließender Chromatographie an Sephadex G 50 wurden 128 mg (52 %) orange-braunes Azoderivat erhalten.

Relative elektrophoretische Beweglichkeit: 0.0 (Cellogel) bzw. - 1.0 (Diskelektrophorese);

$E_{328}^{1\,cm}$ = 10.8 (bei 6.5 g/l, 0.1 m Tris-Puffer, pH 7.5).

Beispiel 65

325 mg nach Beispiel 2 erhaltenes BPTI-Derivat (50 µMol) wurden, wie in Beispiel 50 beschrieben, citraconyliert. Die Citraconylverbindung wurde mit der aus 21 mg 3-Amino-1,2,4-triazol (250 µMol) dargestellten Diazoniumverbindung bei pH 8.0 gekuppelt. Das Reaktionsgemisch wurde analog zu Beispiel 50 aufgearbeitet. Durch Chromatographie an Sephadex(R) G 50 wurden 30 mg (9 %) polymere Anteile und 212 mg (65 %) orange-rotes Azoderivat erhalten.

Relative elektrophoretische Beweglichkeit: 0.0 (Cellogel) bzw. - 1.14 (Diskelektrophorese);

$E_{328}^{1\,cm}$ = 26.7 (bei 6.5 g/l, 0.1 m Tris-Puffer, pH 7.5);
Aminosäureanalyse: vgl. Tab. 1.

Beispiel 66

590 mg (90 µMol) nach Beispiel 19 dargestelltes BPTI-Derivat wurden analog zu Beispiel 50 citraconyliert, und das Citraconylderivat wurde, wie dort beschrieben, mit aus 31.5 mg 3-Amino-1,2,4-triazol hergestellter Diazoniumverbindung gekuppelt. Die nach Abspaltung der Citraconyl-Reste erhaltene Reaktionslösung

wurde an Sephadex G 10 entsalzt. Durch Chromatographie an Sephadex(R) G 50 wurden neben 30 mg polymeren Anteilen 330 mg (56 %) orangefarbene Substanz erhalten.

Relative elektrophoretische Beweglichkeit: - 1.0 (Cellogel) bzw. - 1.08 (Diskelektrophoerese);

$E^{1\,cm}_{328}$ = 4.2 (bei 6.5 g/l, 0.1 m Tris-Puffer, pH 7.8);

Beispiel 67

Aus 350 mg (55 µMol) nach Beispiel 30 erhaltenem BPTI-Derivat wurden analog zu dem in Beispiel 50 beschriebenen Verfahren durch Umsetzen der intermediär dargestellten Citraconylverbindung mit aus 67 mg 3-Amino-1,2,4-triazol dargestellter Diazoniumverbindung 280 mg olivfarbene Substanz (81 %) erhalten.

Relative elektrophoretische Beweglichkeit: - 0.05 (Cellogel) bzw. - 0.59 und - 0.37 (Diskelektrophorese);

$E^{1\,cm}_{320}$ = 49.5 (bei 6.5 g/l, 0.1 m Tris-Puffer, pH 7.5);

Aminosäureanalyse: vgl. Tab. 1.

Beispiel 68

Wie in Beispiel 50 beschrieben, wurden 520 mg des nach Beispiel 18 erhaltenen BPTI-Derivates (80 µMol) citraconyliert, und das Reaktionsprodukt wurde mit aus 67 mg 3-Amino-1,2,4-triazol (800 µMol) nach Beispiel 62 dargestellter Diazoniumverbindung gekuppelt. Man erhielt 395 mg olivfarbene Substanz (76 %).

Relative elektrophoretische Beweglichkeit: - 0.4 (Cellogel) bzw. - 0.9 und - 0.57 (Diskelektrophorese);

$E^{1\,cm}_{318}$ = 53.2 (bei 6.5 g/l, 0.1 m Tris-Puffer, pH 7.5);

Aminosäureanalyse: vgl. Tab. 1.

Beispiel 69

Bei der Verwendung von 5-Amino-tetrazol anstelle von 3-Amino-1,2,4-triazol wurden analog zu Beispiel 68 340 mg braune Substanz (65 %) erhalten.

Relative elektrophoretische Beweglichkeit: - 0.48 (Cellogel) bzw. - 0.40 und - 0.22 (Diskelektrophorese);

$E_{315}^{1\,cm}$ = 16.4 (bei 6.5 g/l, 0.1 m Tris-Puffer, pH 7.5).

Beispiel 70

Aus 260 mg (40 µMol) nach Beispiel 35 erhaltenem BPTI-Derivat und diazotiertem 3-Amino-1,2,4-triazol gewann man analog zu Beispiel 68 205 mg (79 %) orangefarbenes Azoderivat.

Relative elektrophoretische Beweglichkeit: - 0.15 (Cellogel) bzw. - 0.85 (Diskelektrophorese);

$E_{322}^{1\,cm}$ = 15.5 (bei 6.5 g/l, 0.1 m Tris-Puffer, pH 7.5).

Beispiel 71

Durch Kupplung von 520 mg (80 µMol) nach Beispiel 37 erhaltenem BPTI-Derivat mit aus 42 mg 3-Amino-1,2,4-triazol (500 µMol) dargestellter Diazoniumverbindung und weitere Aufarbeitung analog Beispiel 68 wurden 350 mg (67 %) gold-gelbes Azo-BPTI-Derivat erhalten.

Relative elektrophoretische Beweglichkeit: - 0.28 (Cellogel) bzw. - 0.49, - 0.33 und - 0.16 (Diskelektrophorese);

$E_{315}^{1\,cm}$ = 32.8 (bei 6.5 g/l, 0.1 m Tris-Puffer, pH 7.5).

Beispiel 72

Aus 520 mg (80 µMol) nach Beispiel 38 dargestelltem BPTI-Derivat wurden analog zu Beispiel 62 380 mg (73 %) olivenfarbene Azoverbindung gewonnen.

Relative elektrophoretische Beweglichkeit: - 0.32 (Cellogel) bzw. - 0.65, - 0.53 und - 0.35 (Diskelektrophorese);

$E_{320}^{1\,cm}$ = 42.5 (bei 6.5 g/l, 0.1 m Tris-Puffer, pH 7.5);

Aminosäureanalyse: vgl. Tab. 1.

Beispiel 73

325 mg (50 µMol) nach Beispiel 34 erhaltenes BPTI-Derivat wurden analog zu Beispiel 50 mit 3,5-Dichlor-phenyl-diazoniumverbindung zu 273 mg (84 %) orangefarbenem Azo-BPTI-Derivat umgesetzt.

Relative elektrophoretische Beweglichkeit: - 0.65 u. - 0.35 (Cellogel) bzw. - 1.03 u. - 0.85 (Diskelektrophorese);

$E_{323}^{1\,cm}$ = 8.54 (bei 6.5 g/l, 1 m Essigsäure).

Beispiel 74

Analog zu Beispiel 73 wurden aus 325 mg (50 µMol) nach Beispiel 18 erhaltenem BPTI-Derivat 285 mg (87 %) orange-rote Azoverbindung erhalten.

Relative elektrophoretische Beweglichkeit: - 0.80, - 0.29 und 0.0 (Cellogel) bzw. - 0.91 (Diskelektrophorese);

$E_{322}^{1\,cm}$ = 17.8 (bei 6.5 g/l, 0.1 m Tris-Puffer, pH 7.5).

**Beispiel [illegible]**

Aus 260 mg (40 µMol) nach Beispiel 31 erhaltenem BPTI-Derivat wurden analog zu Beispiel 62 255 mg (98 %) hellbraunes Azo-BPTI-Derivat dargestellt.

Relative elektrophoretische Beweglichkeit: - 1.0 (Cellogel) bzw. - 1.0 und - 0.71 (Diskelektrophorese);

$E^{1\,cm}_{320}$ = 24.0 (bei 6.5 g/l, 0.1 m Tris-Puffer, pH 7.5).

**Beispiel [illegible]**

Aus 260 mg (40 µMol) nach Beispiel 32 dargestelltem BPTI-Derivat wurden in Analogie zu Beispiel 62 186 mg (72 %) hellrotes Azoderivat erhalten.

Relative elektrophoretische Beweglichkeit: - 1.0 und 0.0 (Cellogel) bzw. - 0.95 und - 0.74 (Diskelektrophorese);

$E^{1\,cm}_{310}$ = 4.5 (bei 6.5 g/l, 1 m Essigsäure).

**Beispiel 77**

Wie für Beispiel 62 beschrieben, wurden aus 325 mg (50 µMol) nach Beispiel 33 gewonnenem BPTI-Derivat 256 mg (79 %) orangefarbenes Azo-Der[illegible]at erhalten.

Relative elektrophoretische Beweglichkeit: 0.0 Cellogel bzw. - 0.83 und - 0.74 Di[illegible]kelektrophorese);

$E^{1\,cm}_{328}$ = 40.6 (be[illegible] 5.5 g/l, 1 m Essigsäure).

BAD ORIGINAL

Beispiel 78

306 mg nach Beispiel 33 dargestelltes BPTI-Derivat (45 µMol) wurden analog zu Beispiel 62 mit diazotiertem 3-Amino-1,2,4-triazol gekuppelt. Man erhielt 287 mg (94 %) braunes Azo-Derivat.

Relative elektrophoretische Beweglichkeit: - 0.05 (Cellogel) bzw. - 0.95 (Diskelektrophorese);

$E_{320}^{1\,cm}$ = 29.0 (bei 6.5 g/l, 1 m Essigsäure).

Beispiel 79

Analog zu Beispiel 62 wurden aus 650 mg nach Beispiel 45 erhaltenem BPTI-Derivat 563 mg (87 %) olivfarbene Azo-Verbindung dargestellt.

Relative elektrophoretische Beweglichkeit: - 0.44 (Cellogel) bzw. - 0.59, - 0.47 und - 0.31 (Diskelektrophorese);

$E_{318}^{1\,cm}$ = 45.2 (bei 6.5 g/l, 0.1 m Tris-Puffer, pH 7.5).

Beispiel 80

Aus 325 mg nach Beispiel 44 erhaltenem BPTI-Derivat wurden analog zu Beispiel 50 mit diazotiertem 1,2,4- 3-Aminotriazol durch Kuppeln bei pH 3,5 - 4,5 273 mg (84 %) orangefarbenes Azo-BPTI-Derivat gewonnen.

Relative elektrophoretische Beweglichkeit: -0,81 (Diskelektrophorese)

$E_{316}^{1cm}$ = 23.4 (bei 6,5 g/l, 50 proz. Essigsäure)

BAD ORIGINAL

Beispiel 81

Aus 225 mg (35 µMol) nach Beispiel 43 erhaltenem BPTI-Derivat wurden analog zu Beispiel 62 205 mg (91 %) rosafarbenes Azo-Derivat hergestellt.

Relative elektrophoretische Beweglichkeit: - 0.35 und 0.0 (Cellogel) bzw. - 0.42 und - 0.25 (Diskelektrophorese);

$E_{320}^{1\,cm}$ = 7.8 (bei 6.5 g/l, 0.1 m Tris-Puffer, pH 7.5).

Patentansprüche

1. Derivate des BPTI, bei welchen nur ein Teil der Carboxylgruppen des nativen BPTI oder seiner Derivate modifiziert ist, erhältlich durch Umsetzung von BPTI oder von BPTI-Derivaten welche freie Carboxylgruppen enthalten, mit nucleophilen Aminoverbindungen (unter Ausnahme des Glycinäthylesters) in Gegenwart von Carbodiimiden, bei pH-Werten zwischen 3 und 11 in wäßriger Lösung; welche gegebenenfalls auch organische Lösungsmittel und/oder Salze und/oder Denaturierungsmittel enthält, gegebenenfalls Isolierung und weitere Auftrennung der erhaltenen Reaktionsprodukte sowie gegebenenfalls anschließende Desaminierung und/oder gegebenenfalls anschließende Überführung in Azoderivate, deren Tyrosinreste 10 und/oder 21 in ortho-Stellung zur phenolischen Hydroxylgruppe über eine Azogruppe mit einem aromatischen oder heterocyclischen Rest verknüpft sind.

2. Derivate des BPTI gemäß Anspruch 1, dadurch gekennzeichnet, daß sie durch Umsetzung von BPTI oder BPTI-Derivaten welche freue Carboxylgruppen enthalten mit nucleophilen Aminoverbindungen (mit Ausnahme des Glycinäthylesters) in Gegenwart von Carbodiimiden und anschließende Isolierung der Reaktionsprodukte erhalten werden.

3. Derivate des BPTI gemäß Anspruch 1, dadurch gekennzeichnet daß sie nach erfolgter Umsetzung von BPTI oder BPTI-Derivaten welche freie Carboxylgruppen enthalten, mit nucleophilen Aminoverbindungen (mit Ausnahme des Glycinäthylesters) in Gegenwart von Carbodiimiden mit Nitrosylionen liefernden Agenzien oder mit nicht-kuppelnden Diazoniumverbindungen desaminiert worden sind.

BAD ORIGINAL

4. Derivate des BPTI gemäß Anspruch 1, dadurch gekennzeichnet, daß sie nach erfolgter Umsetzung von BPTI oder BPTI-Derivaten welche freie Carboxylgruppen enthalten, mit nucelophilen Aminoverbindungen in der Gegenwart von Carbodiimiden durch Umsetzen mit kupplungsfähigen Diazoniumverbindungen in Azoderivate übergeführt worden sind, deren Tyrosinreste 10 und/oder 21 in ortho-Stellung zur phenolischen Hydroxylgruppe über eine Azogruppe mit einem aromatischen oder heterocyclischen Rest verknüpft sind.

5. Derivate des BPTI gemäß Anspruch 1, dadurch gekennzeichnet, daß sie nach erfolgter Reduktion von Nitro- oder Dinitro-BPTI und Umsetzung mit nucleophilen Aminoverbindungen in Gegenwart von Carbodiimiden mit Nitrosylionen liefernden Verbindungen in Diazoniumderivate umgewandelt worden sind und diese mit Phenolen oder aromatischen Aminen oder Heteroaromaten gekuppelt worden sind.

6. Verfahren zur Herstellung von Derivaten des BPTI, bei welchen nur ein Teil der Carboxylgruppen des nativen BPTI oder seiner Derivate modifiziert ist, dadurch gekennzeichnet, daß man BPTI oder BPTI-Derivate welche freie Carboxylgruppen enthalten, mit nucleophilen Aminoverbindungen (unter Ausnahme des Glycinäthylesters) in Gegenwart von Carbodiimiden bei pH-Werten zwischen 3 und 11 in wäßriger Lösung, welche gegebenenfalls auch organische Lösungsmittel und/oder Salze und/oder Denaturierungsmittel enthält umsetzt, die entstandenen Reaktionsprodukte gegebenenfalls isoliert und gegebenenfalls auftrennt oder die erhaltenen Reaktionsprodukte anschließend mit Nitrosylionen liefernden Agenzien oder mit nicht-kuppelnden

Diazoniumverbindungen desaminiert und/oder gegebenenfalls durch Umsetzung mit kupplungsfähigen Diazoniumverbindungen bei pH-Werten zwischen 1 und 12 in Azoverbindungen überführt und die jeweils entstehenden Reaktionsprodukte nach an sich bekannten Methoden isoliert.

7. Verfahren gemäß Anspruch 6, dadurch gekennzeichnet, daß man Nitro- oder Dinitro-BPTI-Derivate reduziert und nach Umsetzung mit nucleophilen Aminoverbindungen in Gegenwart von Carbodiimiden mit Nitrosylionen liefernden Verbindungen in Diazoniumderivate überführt und diese mit Phenolen, aromatischen Aminen oder Heteroaromaten kuppelt.

8. Arzneimittel gekennzeichnet durch einen Gehalt an BPTI-Derivat oder einem Gemisch von BPTI-Derivaten gemäß Anspruch 1.

9. Verwendung von BPTI-Derivaten gemäß Anspruch 1 bei der Bekämpfung von Krankheiten.

10. Verwendung von BPTI-Derivaten gemäß Anspruch 1 bei der Bekämpfung von Krankheiten, welche auf einer enzymatischen Disfunktion beruhen.

0001774

Europäisches Patentamt

# EUROPÄISCHER RECHERCHENBERICHT

Nummer der Anmeldung

EP 78 101 147.3
- Seite 2 -

| EINSCHLÄGIGE DOKUMENTE | | | KLASSIFIKATION DER ANMELDUNG (Int.Cl.²) |
|---|---|---|---|
| Kategorie | Kennzeichnung des Dokuments mit Angabe, soweit erforderlich, der maßgeblichen Teile | betrifft Anspruch | |
| D,A | Chemical Abstracts Band 70, Nr. 17, 1969<br>Columbus, Ohio, USA<br>J. CHAUVET et al "Role of the carboxyl groups of pancreatic trypsin inhibitor (inhibitor of Kunitz) in its binding with trypsin"<br>Seite 35, Spalte 2, Abstract Nr. 74 578 m<br>& FEBS (Fed. Eur. Biochem. Soc.) Lett.<br>Band 2, Nr. 1, 1968,<br>Seiten 17 bis 19 *<br>---- | 1 | |
| | | | RECHERCHIERTE SACHGEBIETE (Int. Cl.²) |

0001774

Europäisches Patentamt

# EUROPÄISCHER RECHERCHENBERICHT

Nummer der Anmeldung

EP 78 101 147.3

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| P | DE - A - 2 619 246 (BAYER)<br>* Anspruch 4 *<br>-- | [illegible] |
| P | DE - A - 2 654 124 (BAYER)<br>* Anspruch 1 *<br>-- | 4 |
| D | Chemical Abstracts Band 67, Nr. 1, 1967<br>Columbus, Ohio, USA<br>D.G. HOARE et al "A method for the quantitative modification and estimation of carboxylic acid groups in proteins"<br>Seite 146, Spalte 2, abstract Nr. 144[illegible]<br>& J. Biol. Chem. Band 242, Nr. 10, 1967,<br>Seiten 2435 bis 244[illegible]<br>-- | 1 |

./..

**KLASSIFIKATION DER ANMELDUNG (Int.Cl.²)**

C 07 G 7/00
A 61 K 37/64
C 07 G 7/02

**RECHERCHIERTE SACHGEBIETE (Int. Cl.²)**

A 61 K 37/64
C 07 G 7/00
C 07 G 7/02

**KATEGORIE DER GENANNTEN DOKUMENTE**

- X: von besonderer Bedeutung
- A: technologischer Hintergrund
- O: nichtschriftliche Offenbarung
- P: Zwischenliteratur
- T: der Erfindung zugrunde liegende Theorien oder Grundsätze
- E: kollidierende Anmeldung
- D: in der Anmeldung angeführtes Dokument
- L: aus andern Gründen angeführtes Dokument
- &: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Berlin | 11-01-1979 | KNAACK |